(19) Europäisches Patentamt
European Patent Office
Office européen des brevets

(11) EP 1 546 130 B1

(12) EUROPEAN PATENT SPECIFICATION

(45) Date of publication and mention of the grant of the patent:
**01.08.2007 Bulletin 2007/31**

(21) Application number: **03798620.5**

(22) Date of filing: **12.09.2003**

(51) Int Cl.:
***C07D 401/06*** (2006.01) ***C07D 401/14*** (2006.01)
***A61K 31/4545*** (2006.01) ***A61P 1/04*** (2006.01)
***A61P 11/00*** (2006.01) ***A61P 17/00*** (2006.01)
***A61P 19/02*** (2006.01) ***A61P 27/00*** (2006.01)
***A61P 29/00*** (2006.01) ***A61P 37/00*** (2006.01)

(86) International application number:
**PCT/SE2003/001425**

(87) International publication number:
**WO 2004/029041 (08.04.2004 Gazette 2004/15)**

# (54) PIPERIDINE DERIVATIVES FOR USE IN THE TREATMENT OF CHEMOKINE MEDIATED DISEASE STATES

PIPERIDINDERIVATE ZUR VERWENDUNG BEI DER BEHANDLUNG VON DURCH CHEMOKINS VERMITTELTEN LEIDEN

DERIVES DE PIPERIDINE DESTINES AU TRAITEMENT DES ETATS PATHOLOGIQUES MEDIES PAR UNE CHIMIOKINE

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **24.09.2002 SE 0202838**

(43) Date of publication of application:
**29.06.2005 Bulletin 2005/26**

(60) Divisional application:
**07010143.1**

(73) Proprietor: **AstraZeneca AB**
**151 85 Södertälje (SE)**

(72) Inventors:
- **LUCKHURST, Christopher**
**Loughborough, Leicestershire LE11 5RH (GB)**
- **PERRY, Matthew**
**Loughborough, Leicestershire LE11 5RH (GB)**
- **SPRINGTHORPE, Brian**
**Loughborough, Leicestershire LE11 5RH (GB)**

(56) References cited:
**EP-A1- 1 076 055**
**WO-A1-00/00488**
**WO-A1-00/35877**
**WO-A1-01/77101**
**WO-A1-98/01425**
**WO-A1-98/06697**
**WO-A1-02/081449**

EP 1 546 130 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention concerns piperidine derivatives having pharmaceutical activity, to processes for preparing such derivatives, to pharmaceutical compositions comprising such derivatives and to the use of such derivatives as active therapeutic agents.

Pharmaceutically active piperidine derivatives are disclosed in WO99/38514, WO99/04794, WO00/35877, WO 01/77101, WO 00/00488, WO 98/01425, WO 98/06697, WO 02/081449 and EP-A1-1076055.

Histamine is a basic amine, 2-(4-imidazolyl)-ethylamine, and is formed from histidine by histidine decarboxylase. It is found in most tissues of the body, but is present in high concentrations in the lung, skin and in the gastrointestinal tract. At the cellular level inflammatory cells such as mast cells and basophils store large amounts of histamine. It is recognised that the degranulation of mast cells and basophils and the subsequent release of histamine is a fundamental mechanism responsible for the clinical manifestation of an allergic process. Histamine produces its actions by an effect on specific histamine G-protein coupled receptors, which are of three main types, H1, H2 and H3- Histamine H1 antagonists comprise the largest class of medications used in the treatment of patients with allergic disorders, such as rhinitis and urticaria. H1 antagonists are useful in controlling the allergic response by for example blocking the action of histamine on post-capillary venule smooth muscle, resulting in decreased vascular permeability, exudation and oedema. The antagonists also produce blockade of the actions of histamine on the H1 receptors on c-type nociceptive nerve fibres, resulting in decreased itching and sneezing.

Viral infections are known to cause lung inflammation. It has been shown experimentally that the common cold increases mucosal output of eotaxin in the airways. Instillation of eotaxin into the nose can mimic some of the signs and symptoms of a common cold. (See, Greiff L et al Allergy (1999) 54(11) 1204-8 [Experimental common cold increase mucosal output of eotaxin in atopic individuals] and Kawaguchi M et al Int. Arch. Allergy Immunol. (2000) 122 S1 44 [Expression of eotaxin by normal airway epithelial cells after virus A infection].)

Chemokines are chemotactic cytokines that are released by a wide variety of cells to attract macrophages, T cells, eosinophils, basophils and neutrophils to sites of inflammation and also play a rôle in the maturation of cells of the immune system-Chemokines play an important rôle in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C, or $\alpha$) and Cys-Cys (C-C, or $\beta$) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1$\alpha$ and 1$\beta$ (MIP-1$\alpha$ and MIP-1$\beta$.

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those mentioned above.

The present invention provides a compound of formula (I):

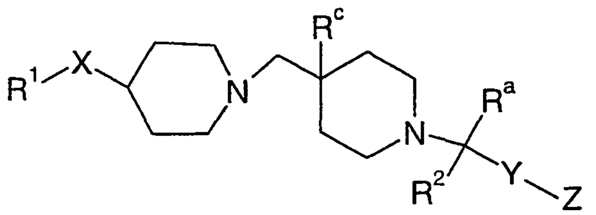

(I)

wherein:

X is $CH_2$, C(O), O, S, S(O), $S(O)_2$ or $NR^3$;
Y is a bond, $C_{1-6}$ alkylene (optionally substituted by $C_{1-4}$ alkyl or phenyl), phenylene (optionally substituted by halogen, hydroxy, $C_{1-4}$ alkyl or $C_{1-4}$ alkoxy) or heterocyclylene (optionally substituted by halogen, hydroxy, $C_{1-4}$ alkyl or $C_{1-4}$ alkoxy);

Z is $CO_2R^b$, $NHS(O)_2CF_3$, $S(O)_2$,OH, $OCH_2CO_2R^b$ or tetrazolyl;
$R^1$ is hydrogen, $C_{1-6}$ alkyl, aryl or heterocyclyl;
$R^2$ is hydrogen, $C_{1-6}$ alkyl, aryl or heterocyclyl;
$R^a$ and $R^b$ are, independently, hydrogen or $C_{1-4}$ alkyl; or when $R^2$ is aryl or heterocyclyl $R^a$ may be $C_{2-3}$ alkylene forming a ring with an ortho position on $R^2$;
$R^c$ is hydrogen or hydroxy;

wherein, unless stated otherwise, the foregoing aryl and heterocyclyl moieties are optionally substituted by: halogen, cyano, nitro, hydroxy, oxo, $S(O)_pR^4$, $OC(O)NR^5R^6$, $NR^7R^8$, $NR^9C(O)R^{10}$, $NR^{11}C(O)NR^{12}R^{13}$, $S(O)_2NR^{14}R^{15}$, $NR^{16}S(O)_2R^{17}$, $C(O)NR^{18}R^{19}$, $C(O)R^{20}$, $CO_2R^{21}$, $NR^{22}CO_2R^{23}$, $C_{1-6}$ alkyl, $CF_3$, $C_{1-6}$ alkoxy($C_{1-6}$)alkyl, $C_{1-6}$ alkoxy, $OCF_3$, $C_{1-6}$ alkoxy($C_{1-6}$)alkoxy, $C_{1-6}$ alkylthio, $C_{2-6}$ alkenyl, $C_{2-6}$ alkynyl, $C_{3-10}$ cycloalkyl (itself optionally substituted by $C_{1-4}$ alkyl or oxo), methylenedioxy, difluoromethylenedioxy, phenyl, phenyl($C_{1-4}$)alkyl, phenoxy, phenylthio, phenyl($C_{1-4}$)alkoxy, heterocyclyl, heterocyclyl($C_{1-4}$)alkyl, heterocyclyloxy or heterocyclyl($C_{1-4}$)alkoxy; wherein any of the immediately foregoing phenyl and heterocyclyl moieties are optionally substituted with halogen, hydroxy, nitro, $S(O)_q$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^5$ and $R^6$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$;
p and q are, independently, 0, 1 or 2;
$R^3$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{18}$, $R^{19}$, $R^{20}$, $R^{21}$ and $R^{22}$ are, independently, hydrogen, $C_{1-6}$ alkyl (optionally substituted by halogen, hydroxy or $C_{3-10}$ cycloalkyl), $CH_2$($C_{2-6}$ alkenyl), phenyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^5$ and $R^6$ below), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^5$ and $R^6$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$) or heterocyclyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^5$ and $R^6$ below), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^5$ and $R^6$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$);
alternatively $NR^5R^6$, $NR^7R^8$, $NR^{12}R^{13}$, $NR^{14}R^{15}$, $NR^{18}R^{19}$, may, independently, form a 4-7 membered heterocyclic ring, azetidine, pyrrolidine, piperidine, azepine, morpholine or piperazine, the latter optionally substituted by $C_{1-4}$ alkyl on the distal nitrogen;
$R^4$, $R^{17}$ and $R^{23}$ are, independently, $C_{1-6}$ alkyl (optionally substituted by halogen, hydroxy or $C_{3-10}$ cycloalkyl), $CH_2$($C_{2-6}$ alkenyl), phenyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^5$ and $R^6$ above), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ above), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^5$ and $R^6$ above), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$) or heterocyclyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^5$ and $R^6$ above), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ above), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^5$ and $R^6$ above), $CO_2H$ $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$);
aryl is phenyl or naphthyl;
heterocyclyl is furyl, thienyl, pyrrolyl, 2,5-dihydropyrrolyl, thiazolyl, pyrazolyl, oxazolyl, isoxazolyl, imidazolyl, piperidinyl, morpholinyl, pyridinyl, dihydropyridinyl, pyrimidinyl, indolyl, 2,3-dihydroindolyl, benzo[b]furyl, benz[b]thienyl, 2,3-dihydrobenz[b]thienyl, indazolyl, benzimidazolyl, benztriazolyl, benzoxazolyl, benzthiazolyl, 2,3-dihydrobenzthiazolyl, 1,2,3-benzothiadiazolyl, an imidazopyridinyl, thieno[3,2-b]pyridin-6-yl 1,2,3-benzoxadiazolyl, 2,1,3-benzothiadiazolyl, benzofurazan, quinoxalinyl, dihydro-1-benzopyryliumyl, 3,4-dihydro-1H-2,1-benzothiazinyl, a pyrazolopyridine, a purine, quinolinyl, isoquinolinyl, dihydroisoquinolinyl, a naphthyridinyl, a dihydro[1,8]naphthyridinyl, a benzothiazinyl, a dihydrobenzothiazinyl, benzo[d]imidazo[2,1-b]thiazol-2-yl or dibenzothiophenyl;
or an N-oxide thereof; or a pharmaceutically acceptable salt thereof; or a solvate thereof.

Certain compounds of the present invention can exist in different isomeric forms (such as enantiomers, diastercorners, geometric isomers or tautomers). The present invention covers all such isomers and mixtures thereof in all proportions.

Suitable salts include acid addition salts such as a hydrochloride, dihydrochloride, hydrobromide, sulfate, phosphate, acetate, diacetate, fumarate, maleate, tartrate, citrate, oxalate, methanesulfonate, ethanesulfonate or *p*-toluencsulfonate. Salts also include metal salts, such as an alkali metal salt (for example a sodium or potassium salt) or an alkaline earth metal salt (for example magnesium or calcium).

The compounds of the invention may exist as solvates (such as hydrates) and the present invention covers all such solvates.

Halogen includes fluorine, chlorine, bromine and iodine. Halogen is, for example, fluorine or chlorine.

Alkyl groups and moieties are straight or branched chain and comprise, for example, 1 to 6 (such as 1 to 4) carbon atoms. Examples of alkyl groups are methyl, ethyl, n-propyl, iso-propyl or tert-butyl.

Alkylene is a straight carbon chain of 1 to 6 carbons, which is optionally substituted. Alkylene includes $CH_2$ or $CH_2CH_2$, and when substituted by alkyl (for example) it can be $CH(CH_3)$ or $CH_2C(CH_3)_2$.

Alkenyl groups comprise, for example, 2 to 6 (such as 2 to 4) carbon atoms. Examples of alkenyl groups are vinyl or allyl.

Alkynyl groups comprise, for example, 2 to 6 (such as 2 to 4) carbon atoms. An example of an alkynyl group is propargyl.

In one embobiment cycloalkyl groups comprise from 3 to 10 (such as 3 to 8, for example 3 to 6) carbon atoms and are mono-, bi or tricyclic. Cycloalkyl is, for example, cyclopropyl, cyclopentyl, cyclohexyl, norbornyl or camphoryl. The cycloalkyl ring is optionally fused to a benzene ring (for example forming a bicyclo[4.2.0]octa-1,3,5-trienyl or indanyl ring system).

In another embodiment cycloalkenyl comprises from 3 to 8 (such as from 3 to 6) carbon atoms and is, for example, monocyclic. Cycloalkenyl is, for example, cyclopentenyl or cyclohexenyl.

Aryl includes phenyl or naphthyl.

Heterocyclyl is an aromatic or non-aromatic 5 or 6 membered ring, optionally fused to one or more other rings, comprising at least one heteroatom selected from the group comprising nitrogen, oxygen and sulfur; or an N-oxide thereof, or an S-oxide or S-dioxide thereof. Heterocyclyl is, for example, furyl, thienyl (also known as thiophenyl), pyrrolyl, 2,5-dihydropyrrolyl, thiazolyl, pyrazolyl, oxazolyl, isoxazolyl, imidazolyl, piperidinyl, morpholinyl, pyridinyl, dihydropyridinyl (for example in a 6-oxo-1,6-dihydro-pyridinyl moiety), pyrimidinyl, indolyl, 2,3-dihydroindolyl, benzo[b]furyl (also known as benzfuryl), benz[b]thienyl (also known as benzthienyl or benzthiophenyl), 2,3-dihydrobenz[b]thienyl (for example in a 1-dioxo-2,3-dihydrobenz[b]thienyl moiety), indazolyl, benzimidazolyl, benztriazolyl, benzoxazolyl, benzthiazolyl (for example in a 1H-benzthiazol-2-one-yl moiety), 2,3-dihydrobenzthiazolyl (for example in a 2,3-dihydrobenzthiazol-2-one-yl moiety), 1,2,3-benzothiadiazolyl, an imidazopyridinyl (such as imidazo[1,2a]pyridinyl), thieno[3,2-b]pyridin-6-yl 1,2,3-benzoxadiazolyl (also known as benzo[1,2,3]thiadiazolyl), 2,1,3-benzothiadiazolyl, benzofurazan (also known as 2,1,3-benzoxadiazolyl), quinoxalinyl, dihydro-1-benzopyryliumyl (for example in a coumarinyl or a chromonyl moiety), 3,4-dihydro-1H-2,1-benzothiazinyl (for example in a 2-dioxo-3,4-dihydro-1H-2,1-benzothiazinyl moiety), a pyrazolopyridine (for example 1H-pyrazolo[3,4-b]pyridinyl), a purine (for example in a 3,7-dihydro-purin-2,6-dione-8-yl moiety), quinolinyl, isoquinolinyl, dihydroisoquinolinyl (for example in a 2H-isoquinolin-1-one-yl moiety), a naphthyridinyl (for example [1,6]naphthyridinyl or [1,8]naphthyridinyl), a dihydro[1,8]naphthyridinyl (for example in a 1H-[1,8]naphthyridin-4-one-yl moiety), a benzothiazinyl, a dihydrobenzothiazinyl (for example in a 4H-benzo[1,4]thiazin-3-one-yl moiety), benzo[d]imidazo[2,1-b]thiazol-2-yl or dibenzothiophenyl (also known as dibenzothienyl); or an N-oxide thereof, or an S-oxide or S-dioxide thereof.

An N-oxide of a compound of formula (I) is, for example, a 1-oxy-[1,4']bipiperidinyl-1'-yl compound.

Phenylene is a phenyl ring joining the carbon to which, *inter alia,* $R^2$ is attached, and the group Z (such as in Example 42 below).

Heterocyclylene is a heterocyclyl ring joining the carbon to which, *inter alia,* $R^2$ is attached, and the group Z (such as in Example 48 below). Heterocyclylene is, for example, pyridyl or oxazolyl.

When $R^2$ is aryl or heterocyclyl and $R^a$ is $C_{2-3}$ alkylene which forms a ring with an ortho position on $R^2$ the resulting compound comprises, for example, an indene ring system. (See, for example, Example 41.)

Phenyl($C_1$-$_4$ alkyl) is, for example, benzyl or 2-phenyleth-l-yl.

Phenyl($C_1$-$_4$ alkoxy) is, for example, benzyloxy or 2-phenyleth-1-yloxy.

Heterocyclyl($C_{1-4}$ alkyl) is, for example, pyridylmethyl or 2-pyridyleth-1-yl.

Heterocyclyl($C_{1-4}$ alkoxy) is, for example, pyridyloxy or 2-pyridyleth-1-yloxy.

In one particular aspect the invention provides a compound of formula (Ia):

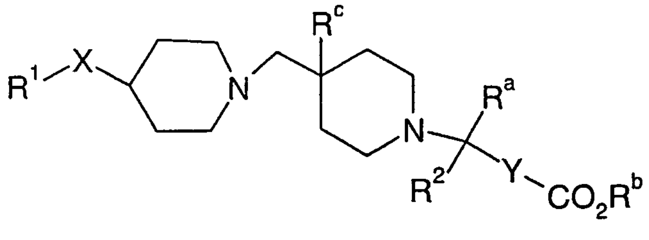

(Ia)

wherein:

X is $CH_2$, C(O), O, S, S(O), $S(O)_2$ or $NR^3$;
Y is a bond, $C_{1-6}$ alkylene (optionally substituted by $C_{1-4}$ alkyl or phenyl) or phenylene (optionally substituted by halogen, hydroxy, $C_{1-4}$ alkyl or $C_{1-4}$ alkoxy);
$R^a$ and $R^b$ are, independently, hydrogen or $C_{1-4}$ alkyl;
$R^c$ is hydrogen or hydroxy;
$R^1$ is hydrogen, $C_{1-6}$ alkyl, aryl or heterocyclyl;
$R^2$ is hydrogen, $C_{1-6}$ alkyl, aryl or heterocyclyl;

wherein, unless stated otherwise, the foregoing aryl and heterocyclyl moieties are optionally substituted by: halogen, cyano, nitro, hydroxy, oxo, $S(O)_pR^4$, $OC(O)NR^5R^6$ , $NR^7R^8$, $NR^9C(O)R^{10}$, $NR^{11}C(O)NR^{12}R^{13}$, $S(O)_2NR^{14}R^{15}$, $NR^{16}S(O)_2R^{17}$, $C(O)NR^{18}R^{19}$, $C(O)R^{20}$, $CO_2R^{21}$, $NR^{22}CO_2R^{23}$ $C_{1-6}$ alkyl, $CF_3$, $C_{1-6}$ alkoxy($C_{1-6}$)alkyl, $C_{1-6}$ alkoxy, $OCF_3$, $C_{1-6}$ alkoxy($C_{1-6}$)alkoxy, $C_{1-6}$ alkylthio, $C_{2-6}$ alkenyl, $C_{2-6}$ alkynyl, $C_{3-10}$ cycloalkyl (itself optionally substituted by $C_{1-4}$ alkyl or oxo), methylenedioxy, difluoromethylenedioxy, phenyl, phenyl($C_{1-4}$)alkyl, phenoxy, phenylthio, phenyl($C_{1-4}$)alkoxy, heterocyclyl, heterocyclyl($C_{1-4}$)alkyl, heterocyclyloxy or heterocyclyl($C_{1-4}$)alkoxy; wherein any of the immediately foregoing phenyl and heterocyclyl moieties are optionally substituted with halogen, hydroxy, nitro, $S(O)_q$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$(and these alkyl groups may join to form a ring as described for $R^5$ and $R^6$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$;
p and q are, independently, 0, 1 or 2;
$R^3$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{18}$, $R^{19}$, $R^{20}$, $R^{21}$ and $R^{22}$ are, independently, hydrogen, $C_{1-6}$ alkyl (optionally substituted by halogen, hydroxy or $C_{3-10}$ cycloalkyl), $CH_2$($C_{2-6}$ alkenyl), phenyl (itself optionally substituted by halogen, hydroxy, nitro, $NH_2$, NH($C_{1-4}$ alkyl), N($C_{1-4}$ alkyl)$_2$, $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^5$ and $R^6$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$) or heterocyclyl (itself optionally substituted by halogen, hydroxy, nitro, $NH_2$, NH($C_{1-4}$ alkyl), N($C_{1-4}$ alkyl)$_2$, $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^5$ and $R^6$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$);
alternatively $NR^5R^6$, $NR^7R^8$, $NR^{12}R^{13}$, $NR^{14}R^{15}$, $NR^{18}R^{19}$, may, independently, form a 4-7 membered heterocyclic ring, azetidine, pyrrolidine, piperidine, azepine, morpholine or piperazine, the latter optionally substituted by $C_{1-4}$ alkyl on the distal nitrogen;
$R^4$, $R^{17}$ and $R^{23}$ are, independently, $C_{1-6}$ alkyl (optionally substituted by halogen, hydroxy or $C_{3-10}$ cycloalkyl), $CH_2$($C_{2-6}$ alkenyl), phenyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^5$ and $R^6$ above), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ above), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^5$ and $R^6$ above), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$) or heterocyclyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^5$ and $R^6$ above), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^5$ and $R^6$ above), cyano, $C_{1-4}$ alkyl, $C_{,1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^5$ and $R^6$ above), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$);

or an N-oxide thereof; or a pharmaceutically acceptable salt thereof; or a solvate thereof.

In another aspect the invention provides a compound wherein X is O.

In yet another aspect $R^1$ is phenyl optionally substituted (for example independently mono- or di-substituted) with halogen (for example chlorine or fluorine), $C_{1-4}$ alkyl (for example methyl) or $C_{1-4}$ alkoxy (for example methoxy).

In a further aspect $R^1$ is phenyl optionally substituted (for example with one, two or three of the same or different) with fluorine, chlorine, $C_{1-4}$ alkyl (for example methyl) or $C_{1-4}$ alkoxy (for example methoxy). In a still further aspect $R^1$ is phenyl substituted by one, two or three (for example two or three) substituents independently selected from: fluorine, chlorine and methyl. For example $R^1$ is 3,4-dichlorophenyl, 2,4-dichloro-3-methylphenyl, 3,4-dichloro-2-methylphenyl, 2,4-dichlorophenyl, 4-chloro-2-methylphenyl or 2-chloro-4-fluorophenyl.

In another aspect $R^a$ is hydrogen.

In another aspect $R^b$ is hydrogen or methyl.

In another aspect $R^c$ is hydrogen.

In a further aspect $R^2$ is unsubstituted phenyl or naphthyl, mono-, di- or trisubstituted phenyl or naphthyl or mono-substituted heterocyclyl, the substituents being chosen from those described above.

Heterocyclyl is, for example, pyrimidinyl or pyridinyl. In a further aspect of the invention heterocyclyl is optionally substituted by $C_{1-4}$ alkyl or $C_{1-4}$ alkoxy.

In another aspect $R^2$ is hydrogen or phenyl optionally substituted by: halogen (for example fluoro), $C_{1-6}$ alkyl, $C_{1-6}$ alkoxy or ($C_{1-6}$ alkyl)C(O)NH.

In a further aspect the present invention provides a compound of formula (I) wherein X is O; $R^1$ is phenyl optionally substituted by halogen (for example chlorine) or $C_{1-4}$ alkyl (for example methyl); and $R^a$, $R^b$, $R^c$ and $R^2$ is as defined above.

In a still further aspect the present invention provides a compound wherein Y is a bond or alkylene (optionally substituted by $C_{1-4}$ alkyl); $R^a$ is hydrogen; and, $R^2$ is hydrogen, $C_{1-6}$ alkyl, phenyl (optionally substituted by halogen, $C_{1-4}$ alkyl, $C_{1-4}$ alkoxy or NHC(O)($C_{1-4}$ alkyl)) or heterocyclyl (optionally substituted by halogen, $C_{1-4}$ alkyl or $C_{1-4}$ alkoxy).

In another aspect the present invention provides a compound wherein Y is phenylene (optionally substituted by halogen, $C_{1-4}$ alkyl or $C_{1-4}$ alkoxy) or heterocyclylene (optionally substituted by halogen, $C_{1-4}$ alkyl or $C_{1-4}$ alkoxy); $R^a$ is hydrogen; and $R^2$ is hydrogen or $C_{1-4}$ alkyl.

When Z is tetrazolyl it is, for example, tetrazol-5-yl. In yet another aspect of the invention Z is $CO_2R^b$, wherein $R^b$ is hydrogen or $C_{1-4}$ alkyl (for example methyl).

The compounds of the invention can be prepared by adaptation of methods known in the art, by adaptation of the Examples given below or by using or adapting the methods in Scheme 1 {in which EDCI is ethyl dimethylaminopropyl carbodiimide; HOBT is 1-hydroxybenzotriazole hydrate; and DMAP is *N,N*-dimethylaminopyridine}.

A compound of formula (I), for example wherein $R^a$ is hydrogen and Z is $CO_2R^b$, can be prepared by coupling a compound of formula (II):

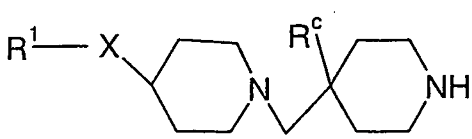

(II)

with a compound of formula (III):

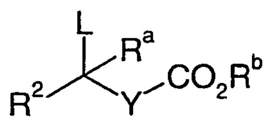

(III)

wherein L is a suitable leaving group (such as halogen (such as chloro or bromo), $C_{1-6}$ alkylsulfonyl (such as mesylate) or tosylate) and the coupling can be carried out in a suitable solvent (such as water or N,N-dimethylformamide) at ambient temperature.

Alternatively, a compound of formula (I), wherein $R^a$ is hydrogen and Z is $CO_2R^b$, can be prepared by reductive amination of a compound (II) with a compound of formula (IV):

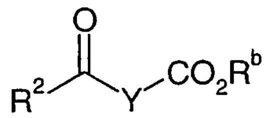

(IV)

wherein $R^b$ is $C_{1-4}$ alkyl, in the presence of $NaBH(OAc)_3$ and acetic acid, or $NaBH_3CN$ in a suitable solvent (such as tetrahydrofuran), optionally followed by hydrolysis of the ester group.

Alternatively, a compound of formula (I), wherein Y is a bond, $R^a$ and $R^b$ are both hydrogen and Z is $CO_2H$ can be prepared by a three component coupling of a compound of formula (II) with compounds of formula (V) and (VI):

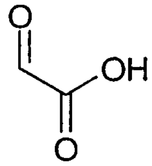

(V)

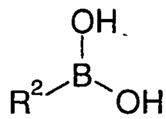

(VI)

in a suitable solvent (such as a $C_{1-6}$ aliphatic alcohol (for example ethanol)) at a suitable elevated temperature (for example reflux; such as 60-100°C).

A compound of formula (II) can be prepared by deprotecting a compound of formula (VII):

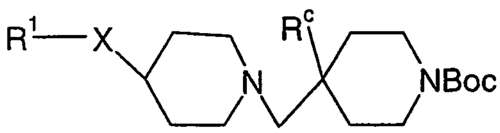

(VII)

for example using trifluoroacetic acid in a suitable solvent (such as dichloromethane) or using a source of hydrogen chloride in a suitable solvent (such as dioxane).

A compound of formula (VII), wherein $R^c$ is hydrogen, can be prepared by reacting a compound of formula (VIII):

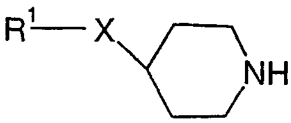

(VIII)

with a compound of formula (IX):

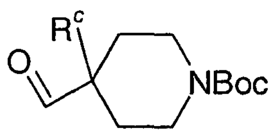

(IX)

in the presence of $NaBH(OAc)_3$ and acetic acid, in a suitable solvent (such as tetrahydrofuran or dichloromethane).

A compound of formula (VII), wherein $R^c$ is hydroxy, can be prepared by reacting a compound of formula (VIII) with a compound of formula (X):

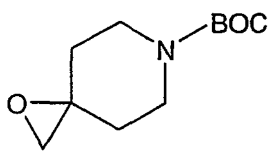

(X)

in a suitable solvent (such as a $C_{1-6}$ aliphatic alcohol, for example ethanol) at room temperature.

A compound of formula (I), wherein Y is a bond and Z is $CO_2H$, can be prepared by performing a nitrile hydrolysis on a compound of formula (XI):

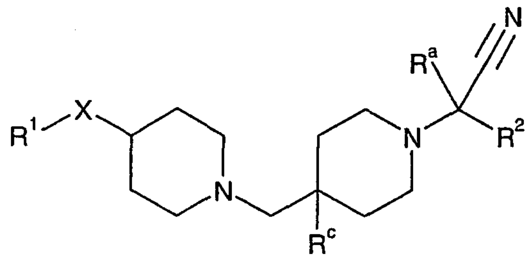
(XI)

Such a hydrolysis can be carried out by refluxing a mixture of hydrochloric acid and ethanol; or by adding $MeSO_3H$, water and hydrochloric acid and then refluxing the mixture.

A compound of formula (XI) can be used to form a compound of formula (I) wherein Z is tetrazol-5-yl by reacting it with $(CH_3)_3SiN_3$ and $(Bu_3Sn)_2O$ at an elevated temperature (for example in toluene at reflux).

A compound of formula (XI) can be reduced to form a compound of formula (XII):

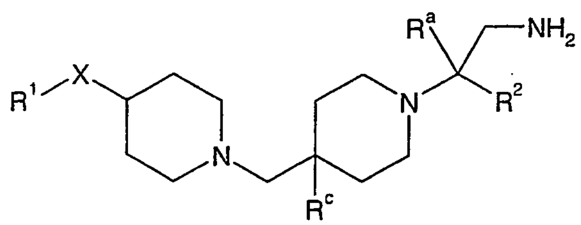
(XII)

using sodium borohydride and cobalt (II) chloride in methanol. A compound of formula (XII) can then be reacted with triflic anhydride at a reduced temperature (for example -78°C in dichloromethane) to form the corresponding compound where Z is $NHS(O)_2CF_3$.

A compound of formula (XI) can be prepared by reacting a compound of formula (II) with $R^aR^2C(O)$ and titanium isopropoxide ($Ti(OiPr)_4$), followed by $Et_2AlCN$. Longer chain variants of the compound of formula (XI) can be made by reacting a compound of formula (II) with: a compound Hal-$(CH_2)_n$CN in the presence of a base (such as potassium carbonate) in acetone; or $CH_2$=CH-CN in the presence of a base (such as potassium carbonate) in acetone; wherein Hal is chlorine, bromine or iodine.

The preparation of various intermediates can be found in WO00/66559 and WO01/77101; alternatively they can be prepared by using or adapting literature methods.

Compounds of formula (III) to (IX) can be prepared by using or adapting methods described in the art. The preparation of various phenoxy piperidines is described in WO 01/77101.

A compound of formula (I), wherein Y is $CHR^d$; $R^d$ is hydrogen, $C_{1-4}$ alkyl or phenyl; and Z is $CO_2R^b$ , can be prepared by reacting a compound of formula (II) with an alkene of formula $R^2R^aC{=}CHR^dCO_2R^b$ in a suitable solvent, such as ethanol, at a suitable elevated temperature, such as 50-100°C.

A compound of formula (I), wherein $R^a$ is hydrogen, Y is $CH_2$ and Z is $CO_2R^b$, can be prepared by reacting a compound of formula (II) with an alkyne of formula $R^2C{\equiv}CCO_2R^b$ in a suitable solvent, such as ethanol, at a suitable elevated temperature, such as 50-100°C; and then reducing the alkene product so formed (for example by catalytic hydrogenation).

A compound of formula (1), wherein $R^2$ and $R^a$ are hydrogen, Y is phenylene (optionally substituted by halogen, hydroxy, $C_{1-4}$ alkyl or $C_{1-4}$ alkoxy) and Z is $CO_2R^b$, can be prepared by reacting a compound of formula (II) with a benzyl bromide of formula $BrCH_2$-Y-$CO_2R^b$ in the presence of diisopropylethylamine (DIPEA), in a suitable solvent (such as acetonitrile) and at ambient temperature (such as in the range 10-30°C).

Alternatively, a compound of formula (I), wherein $R^2$ and $R^a$ are hydrogen, Y is phenylene (optionally substituted by halogen, hydroxy, $C_{1-4}$ alkyl or $C_{1-4}$ alkoxy) and Z is $CO_2R^b$, can be prepared by reacting a compound of formula (II) with a benzaldehyde of formula (O)HC-Y-$CO_2R^b$ wherein $R^b$ is $C_{1-4}$ alkyl, in the presence of $NaBH(OAc)_3$ and acetic acid, in a suitable solvent (such as tetrahydrofuran), optionally followed by hydrolysis of the ester group.

Compounds of formula (I) wherein $R^2$, and $R^a$ are both hydrogen; Y is $CH_2$; and Z is $CO_2R^b$ can be prepared by a Michael addition of $CH_2=CH-CO_2R^b$ on a compound of formula (II).

In another aspect the present invention provides processes for the preparation of compounds of formula (I) or (Ia).

The compounds of the invention have activity as pharmaceuticals, in particular as modulators of chemokine receptor (such as CCR3) activity, and may be used in the treatment of autoimmune, inflammatory, proliferative or hyperproliferative diseases, or immunologically-mediated diseases (including rejection of transplanted organs or tissues and Acquired Immunodeficiency Syndrome (AIDS)).

Examples of these conditions are:

(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}; bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis; sarcoidosis; farmer's lung and related diseases; nasal polyposis; fibroid lung, idiopathic interstitial pneumonia, antitussive activity, treatment of chronic cough associated with inflammatory conditions of the airways or iatrogenic induced cough;
(2) (bone and joints) arthrides including rheumatic, infectious, autoimmune, seronegative spondyloarthropathies (such as ankylosing spondylitis, psoriatic arthritis or Reiter's disease), Behçet's disease, Sjogren's syndrome or systemic sclerosis;
(3) (skin and eyes) psoriasis, atopic dermatitis, contact dermatitis or other eczmatous dermitides, seborrhoetic dermatitis, lichen planus, phemphigus, bullous phemphigus, epidermolysis bullosa, urticaria, angiodermas, vasculitides erythemas, cutaneous cosinophilias, uveitis, alopecia areata, corneal ulcer or vernal conjunctivitis;
(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);
(5) (Allograft rejection) acute and chronic following, for example, transplantation of kidney, heart, liver, lung, bone marrow, skin or cornea; or chronic graft versus host disease; and/or
(6) (other tissues or diseases) Alzheimer's disease, multiple sclerosis, atherosclerosis, Acquired Immunodeficiency Syndrome (AIDS), lupus disorders (such as lupus erythematosus or systemic lupus), erythematosus, Hashimoto's thyroiditis, myasthenia gravis, type I diabetes, nephrotic syndrome, eosinophilia fascitis, hyper IgE syndrome, leprosy (such as lepromatous leprosy), peridontal disease, Sezary syndrome, idiopathic thrombocytopenia pupura or disorders of the menstrual cycle.

The compounds of formula (I) or (Ia) or a pharmaceutically acceptable salt thereof or a solvate thereof, are also H1 antagonists (and can, therefore, be used in the treatment of allergic disorders): and may also be used to control a sign and/or symptom of what is commonly referred to as a cold (for example a sign and/or symptom of a common cold or influenza or other associated respiratory virus infection).

The invention also provides a compound of the formula (I) or (Ia), or a pharmaceutically acceptable salt thereof or a solvate thereof, for use in therapy.

In another aspect the invention provides the use of a compound of formula (I) or (Ia), or a pharmaceutically acceptable salt thereof or a solvate thereof, in the manufacture of a medicament for use in therapy (for example modulating chemokine receptor activity (such as CCR3 receptor activity), antagonising H1 or treating a sign and/or symptom of what is commonly referred to as a cold).

The invention further provides the use of a compound of formula (I) or (Ia), or a pharmaceutically acceptable salt thereof, in the manufacture of a medicament for use in the treatment of:

(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}: bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis; sarcoidosis; farmer's lung and related diseases; nasal polyposis; fibroid lung, idiopathic interstitial pneumonia, antitussive activity, treatment of chronic cough associated with inflammatory conditions of the airways or iatrogenic induced cough;

(2) (bone and joints) arthrides including rheumatic, infectious, autoimmune, seronegative spondyloarthropathies (such as ankylosing spondylitis, psoriatic arthritis or Reiter's disease), Behcet's disease, Sjogren's syndrome or systemic sclerosis;

(3) (skin and eyes) psoriasis, atopic dermatitis, contact dermatitis or other eczmatous dermitides, seborrhoetic dermatitis, lichen planus, phemphigus, bullous phemphigus, epidermolysis bullosa, urticaria, angiodermas, vasculitides erythemas, cutaneous eosinophilias, uveitis, alopecia areata, corneal ulcer or vernal conjunctivitis;

(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);

(5) (Allograft rejection) acute and chronic following, for example, transplantation of kidney, heart, liver, lung, bone marrow, skin or cornea; or chronic graft versus host disease; and/or

(6) (other tissues or diseases) Alzheimer's disease, multiple sclerosis, atherosclerosis, Acquired Immunodeficiency Syndrome (AIDS), lupus disorders (such as lupus erythematosus or systemic lupus), erythematosus, Hashimoto's thyroiditis, myasthenia gravis, type I diabetes, nephrotic syndrome, eosinophilia fascitis, hyper IgE syndrome, leprosy (such as lepromatous leprosy), Peridontal disease, sezary syndrome, idiopathic thrombocytopenia pupura or disorders of the menstrual cycle;

in a mammal (for example man).

In a further aspect the invention provides a compound of formula (I) or (Ia), or a pharmaceutically acceptable salt thereof, for use in the treatment of asthma {such as bronchial, allergic, intrinsic, extrinsic or dust asthma, particularly chronic or inveterate asthma (for example late asthma or airways hyper-responsiveness)}; or rhinitis {including acute, allergic, atrophic or chronic rhinitis, such as rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis}.

In a still further aspect a compound of formula (I) or (Ia), or a pharmaceutically acceptable salt thereof, is useful in the treatment of asthma.

The present invention also provides a the use of a compound of formula (I) or (Ia), or a pharmaceutically acceptable salt thereof, in the manufacture of a medicament for use in the treatment of asthma {such as bronchial, allergic, intrinsic, extrinsic or dust asthma, particularly chronic or inveterate asthma (for example late asthma or airways hyper-responsiveness)}; or rhinitis {including acute, allergic, atrophic or chronic rhinitis, such as rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis}.

In order to use a compound of the invention, or a pharmaceutically acceptable salt thereof or solvate thereof, for the therapeutic treatment of a mammal, such as man, said ingredient is normally formulated in accordance with standard pharmaceutical practice as a pharmaceutical composition. Therefore in another aspect the present invention provides a pharmaceutical composition which comprises a compound of the formula (I) or (Ia), or a pharmaceutically acceptable salt thereof or a solvate thereof (active ingredient), and a pharmaceutically acceptable adjuvant, diluent or carrier.

In a further aspect the present invention provides a process for the preparation of said composition which comprises mixing active ingredient with a pharmaceutically acceptable adjuvant, diluent or carrier. Depending on the mode of administration, the pharmaceutical composition will, for example, comprise from 0.05 to 99 %w (per cent by weight), such as from 0.05 to 80 %w, for example from 0.10 to 70 %w, such as from 0.10 to 50 %w, of active ingredient, all percentages by weight being based on total composition.

The pharmaceutical compositions of this invention may be administered in standard manner for the disease condition that it is desired to treat, for example by topical (such as to the lung and/or airways or to the skin), oral, rectal or parenteral administration. For these purposes the compounds of this invention may be formulated by means known in the art. A suitable pharmaceutical composition of this invention is one suitable for oral administration in unit dosage form, for example a tablet or capsule which contains between 0.1 mg and 1g of active ingredient.

Each patient may receive, for example, a dose of 0.01 1 $mgkg^{-1}$ to 100 $mgkg^{-1}$, such as in the range of 0.1 $mgkg^{-1}$ to 20 $mgkg^{-1}$, of the active ingredient administered, for example, 1 to 4 times per day.

The invention will now be illustrated by the following non-limiting examples in which, unless stated otherwise:

(i) when given, $^{1}H$ NMR data is quoted and is in the form of delta values for major diagnostic protons, given in parts per million (ppm) relative to tetramethylsilane (TMS) as an internal standard, determined at 300MHz or 400MHz using perdeuterio DMSO-D6 ($CD_3SOCD_3$) or $CDCl_3$ as the solvent unless otherwise stated;

(ii) mass spectra (MS) were run with an electron energy of 70 electron volts in the chemical ionisation (CI) mode using a direct exposure probe; where indicated ionisation was effected by electron impact (EI) or fast atom bombardment (FAB); where values for m/z are given, generally only ions which indicate the parent mass are reported, and unless otherwise stated the mass ion quoted is the positive mass ion - $(M+H)^+$;
(iii) the title and sub-title compounds of the examples and methods were named using the index name program from Advanced Chemistry Development Inc;
(iv) unless stated otherwise, reverse phase HPLC was conducted using a Symmetry™, NovaPak™ or Xerra™ reverse phase silica column; and
(v) the following abbreviations are used:

| | |
|---|---|
| Boc or BOC | tert-butoxycarbonyl |
| HPLC | high pressure liquid chromatography |
| DIPEA | Diisopropylethylamine |
| NMP | N-methylpyrrolidone |

| | |
|---|---|
| DMSO | dimethylsulfoxide |
| aq | aqueous |
| THF | tetrahydrofuran |
| MeCN | acetonitrile |

INTERMEDIATE 1

4-(3,4-Dichlorophenoxy)-1-(4-piperidinylmethyl)-piperidine

a) 1,1 -Dimethylethyl 4-[[4-(3,4-dichlorophenoxy)-1-piperidinyl]methyl]-1-piperidinecarboxylate

4-(3,4-Dichlorophenoxy)piperidine (1.27 g) was dissolved in THF (20 mL); acetic acid (0.5 mL) and 1,1-dimethylethyl 4-formyl-1-piperidinecarboxylate (1.43 g) were added to the solution. The reaction mixture was stirred at room temperature for 30 min then sodium triacetoxyborohydride (1.53 g) was added and the mixture was stirred at room temperature overnight. The reaction mixture was poured into 2M sodium hydroxide solution (50 mL) and product was extracted with ether. The ether was washed with brine, dried, filtered and evaporated. Crude material was purified by flash chromatography (eluting with 979 : 20:1 dichloromethane: methanol: aqueous ammonia) to give the subtitle compound (2.15 g).
MS 443/445 $[M+H]^+$ (ES+)
$^1H$ NMR $\delta_{(CDCl3)}$ 1.06 (2H, ddd), 1.45 (9H, s), 1.61 - 1.82 (5H, m), 1.92 - 1.98 (2H, m), 2.16 - 2.27 (4H, m), 2.65 - 2.73 (4H, m), 4.08 (2H, d), 4.25 (1H, dq), 6.75 (1H, dd), 6.99 (1H, d), 7.30 (1H, d)

b) 4-(3,4-dichlorophenoxy)-1-(4-piperidinylmethyl)-piperidine

1,1-Dimethylethyl 4-[[4-(3,4-dichlorophenoxy)-1-piperidinyl]methyl]-1-piperidinecarboxylate (1.0 g) was added to a mixture of 20% TFA in dichloromethane (20 mL) and the mixture was stirred at room temperature for 1h. Solvent was removed by evaporation and 2M sodium hydroxide solution (25 mL) was added to the residue. Product was extracted with ethyl acetate. The organic phase was washed with brine, dried, filtered and evaporated to give the title compound (0.5 g).
MS 343/345 $[M+H]^+$ (ES+)
$^1H$ NMR $\delta_{(CDCl3)}$ 1.10 (2H, qd), 1.60 (1H, qquintet), 1.73- 1.83 (4H, m), 1.90 - 2.01 (2H, m), 2.16 - 2.26 (4H, m), 2.55 - 2.70 (4H, m), 3.09 (2H, d), 4.24 (1H, dquintet), 6.75 (1H, dd), 6.99 (1H, d), 7.27 (1H, d)

The following intermediates were prepared analogously from the appropriate aryloxy piperidine:

| Intermediate | Name | M+H | $^1H$ NMR |
|---|---|---|---|
| 2 | 4-(2,4-Dichloro-3-methylphenoxy)-1-(4-piperidinylmethyl)-piperidine | 357/359 | $\delta_{(CDCl3)}$ 1.13 - 1 .27 (2H, m), 1.57 - 1.70 (1H, m), 1.76 - 2.00 (2H, m), 2.16 - 2.32 (4H, m), 2.46 (3H, s), 2.60 - 2.99 (8H, m), 3.16 (2H, d), 4.31 (1H, quintet), 6.75 (1H, d), 7.18 (1H, d) |

(continued)

| Intermediate | Name | M+H | $^1$H NMR |
|---|---|---|---|
| 3 | 4-(4-Chloro-2-methylphenoxy)-1-(4-piperidinylmethyl)-piperidine | 323/325 | $\delta_{(CDCl3)}$ 1.08-1.21 (2H, m), 1.56 - 1.68 (1H, m), 1.73- 1.86 (4H, m), 1.90 - 1.99 (2H, m), 2.16 - 2.31 (7H, m), 2.57 - 2.69 (4H, m), 3.12 (2H, d), 4.23 - 4.31 (1H, m), 6.74 (1H, d), 7.06 (1H, dd), 7.11 (1H, d) |
| 4 | 4-(2,4-Dichlorophenoxy)-1-(4-piperidinylmethyl)-piperidine | 343/345 | |
| 5 | 4-(3,4-Dichloro-2-methylphenoxy)-1-(4-piperidinylmethyl)-piperidine | 357/359 | $\delta_{(CD3OD)}$ 1.10 - 1.22 (2H, m), 1.66 - 1.85 (5H, m), 1.94 - 2.04 (2H, m), 2.22 (2H, d), 2.31 (3H, s), 2.32 - 2.41 (2H, m), 2.59 - 2.72 (4H, m), 3.08 (2H, d), 4.38 - 4.46 (1H, m), 6.91 (1H, d), 7.27 (1H, d) |

INTERMEDIATE 6

4-(3-Chloro-4-fluoro-phenoxy)-piperidine

DEAD (0.43 mL) was added to a solution of triphenylphosphine (0.72 g), 3-chloro-4-fluorophenol (0.403 g) and 4-hydroxy-piperidine-1-carboxylic acid tert-butyl ester (0.5 g) in THF at RT. The resulting mixture was stirred overnight, HCl in dioxan (2 mL of 4M) was added and the mixture stirred at RT overnight. The mixture was then evaporated to dryness and triethylamine (5 mL) was added. The mixture was evaporated and the residue was dissolved in methanol (10 mL), placed onto a SCX cartridge (Varian, 10 g, SCX cartridge available from International Sorbent Technology Isolute® Flash SCX-2) and eluted: first with methanol then with 10%$NH_3$ in methanol. The basic fractions were combined and evaporated to give the product as an oil (0.6 g).
$^1$H NMR $\delta$($_{DMSO-D6}$) 1.34 - 1.46 (2H, m), 1.83 - 1.91 (2H, m), 2.53 - 2.59 (2H, m), 2.87 - 2.96 (2H, m), 3.22 - 3.39 (1H, m), 4.39 (1H, septet), 6.92 - 6.98 (1H, m), 7.17 - 7.20 (1H, m), 7.30 (1H, t).

The following intermediate was prepared in similar manner to intermediate 6

| Intermediate | name | M+H |
|---|---|---|
| 7 | 4-(3,4-Dichloro-2-methylphenoxy)-piperidine | 260/262 |

INTERMEDIATE 8

4-[[4-(3,4-Dichlorophenoxy)-1-piperidinyl]methyl]- 4-piperidinol

a) 1,1-Dimethylethyl 4-[[4-(3,4-dichlorophenoxy)-1-piperidinyl]methyl]-4-hydroxy-1-piperidinecarboxylate

A solution of 4-(3,4-dichlorophenoxy)-piperidine (5.2 g) and 1,1-dimethylethyl 1-oxa-6-azaspiro[2.5]octane-6-carboxylate (4.1 g) in ethanol (50 mL) was stirred at room temperature for 18 hours and then at 60°C for 18 hours. The solvent was evaporated to leave 9.5 g of a pale yellow oil. Flash chromatography (dichloromethane then dichloromethane : 7M ammonia in methanol 95:5) gave the subtitle compound (8.48 g).
MS $[M+H]^+$ (ES+) 459/461
$^1$H NMR $\delta_{(CDCl3)}$ 1.35 - 1.63 (4H, m), 1.46 (9H, s), 1.73 - 1.86 (2H, m), 1.89 - 2.01 (2H, m), 2.34 (2H, s), 2.49 - 2.59 (2H, m), 2.79 - 2.89 (2H, m), 3.07 - 3.24 (2H, m), 3.79 - 3.93 (2H, m), 4.22 - 4.32 (1H, m), 6.75 (1H, dd), 6.99 (1H, d), 7.30 (1H, d)

b) 4-[[4-(3,4-Dichlorophenoxy)-1-piperidinyl]methyl]-4-piperidinol

To a solution of 1,1-dimethylethyl 4-[[4-(3,4-dichlorophenoxy)-1-piperidinyl]methyl]-4-hydroxy-1-piperidinecarboxylate (5 g) in dichloromethane (50 mL) was added trifluoroacetic acid (5 mL) and the solution was stirred for 12 hours. Sodium hydroxide solution (1M) was added to give an alkaline solution, this was then extracted thrice with dichloromethane. The pooled organic phase was subsequently washed with water, dried, filtered and evaporated to give the title compound (3.5 g).

MS $[M+H]^+$ (ES+) 359/361

$^1H$ NMR $\delta_{(CDCl3)}$ 1.57 - 1.66 (4H, m), 1.69 - 1.84 (2H, m), 1.93 - 2.04 (2H, m), 2.36 (2H, s), 2.47 - 2.58 (2H, m), 2.82 - 2.92 (4H, m), 2.96 - 3.07 (2H, m), 4.32 - 4.41 (1H, m), 6.89 (1H, dd), 7.09 (1H, d), 7.38 (1H, d)

INTERMEDIATE 9

4-[[4-(3,4-Dichlorophenoxy)-1-piperidinyl]methyl]-1,2-cyclopentanediol

a) 4-(3,4-Dichlorophenoxy)-1-[1-oxo-2-(2-propenyl)-4-pentenyl]-piperidine

A solution of 4-(3,4-dichlorophenoxy)-piperidine (5.25 g) in dichloromethane (80 mL) was added to a solution of EDCI (2.45 g), HOBT (1.77 g) and DMAP (0.44 g) in dichloromethane (100 mL). A solution of 2-(2-propenyl)- 4-pentenoic acid (1.81 g) in dichloromethane (5 mL) was added and the solution was stirred for 60 h. The reaction mixture was poured onto water. The mixture was separated and the aqueous phase was extracted twice with dichloromethane. The organic phases were washed with brine, dried, filtered and evaporated to give an oil. Chromatography of the oil (eluting dichloromethane, then 49:1 dichloromethane : methanol) gave the subtitle compound (3.40 g).

MS $[M+H]^+$ (ES+) 368/342

$^1H$ NMR $\delta_{(CDCL3)}$ 5.69 - 5.83 (2H, m), 5.00 - 5.11 (4H, m), 4.46 - 4.52 (1H, m), 3.62 - 3.85 (3H, m), 3.43 - 3.53 (1H, m), 2.76 - 2.87 (1H, m), 2.37 - 2.47 (2H, m), 2.17 - 2.27 (2H, m), 1.70 - 1.99 (4H, m), 6.77 (1H, dd), 7.01 (1H, d), 7.33 (1H, d)

b) 1-(3-Cyclopenten-1-ylcarbonyl)-4-(3,4-dichlorophenoxy)-piperidine

Nitrogen was bubbled through solution of 4-(3,4-dichlorophenoxy)-1-[1-oxo-2-(2-propenyl)-4-pentenyl]-piperidine (1.45 g) in dichloromethane (20 mL) for 10 min. with sonication (cleaning bath). Grubbs' catalyst (89 mg) was added and the solution was stirred for 16 h. Water was added and the phases were separated. The aqueous phase was extracted twice with dichloromethane, the organics were dried, filtered and concentrated to give the subtitle compound as a green oil (1.60 g)

MS $[M+H]^+$ (ES+) 340/342

$^1H$ NMR $\delta_{(CDCL3)}$ 4.47 - 4.53 (1H, m), 5.67 (2H, s), 7.33 (1H, d), 6.78 (1H, dd), 7.02 (1H, d), 3.62 - 3.84 (3H, m), 3 .44 - 3.52 (1H, m), 3.33 (1H, d), 2.68 - 2.77 (2H, m), 2.54 - 2.64 (2H, m), 1.88 - 1.99 (2H, m), 1.73 - 1.86 (2H, m)

c) *cis* and *trans* 4-(3,4-Dichlorophenoxy)-1-[(3,4-dihydroxycyclopentyl)carbonyl]-piperidine

1-(3-Cyclopenten-1-ylcarbonyl)-4-(3,4-dichlorophenoxy)-piperidine (1.45 g) was dissolved in acetone (30 mL) and water (20 mL). Osmium tetroxide (1 mL of 2.5% solution in t-butanol) was added and the solution was stirred for 5 days. The reaction mixture was poured onto a solution of sodium metabisulfite. The mixture was extracted thrice with dichloromethane, the organic extracts were washed with brine, dried, filtered and evaporated to give an oil. Chromatography (eluting dichloromethane : methanol 24:1 to 37 : 3) gave the title compound as two compounds (0.31 g and 0.71 g).

MS $[M+H]^+$ (ES+) 374/376

Minor isomer $^1H$ NMR $\delta_{(CDCL3)}$ 1.79- 1.98 (6H, m), 2.12 - 2.22 (2H, m), 3.23 (1H, tt), 3.49 - 3.56 (1H, m), 3.65 - 3.79 (3H, m), 3.93 (1H, d), 3.99 - 4.08 (3H, m), 4.53 (1H, tt), 6.77 (1H, dd), 7.02 (1H, d), 7.34 (1H, d)

Major isomer $^1H$ NMR $\delta_{(CDCL3)}$ 1.73- 1.86 (2H, m), 1.86 - 2.00 (4H, m), 2.07 - 2.16 (2H, m), 2.50 - 2.60 (2H, m), 3.39 (1H, tt), 3.42 - 3.48 (1H, m), 3.61 - 3.78 (3H, m), 4.22 - 4.27 (2H, m), 4.47 - 4.53 (1H. m), 6.77 (1H, dd), 7.01 (1H, d), 7.33 (1H, d)

d) 4-[[4-(3,4-Dichlorophenoxy)-1-piperidinyl]methyl]-1,2-cyclopentanediol

Borane solution (16 mL of 1M in THF) was added to 4-(3,4-dichlorophenoxy)-1-[(3,4-dihydroxycyclopentyl) carbonyl]-piperidine (major isomer, 0.71 g) and the resulting solution was heated to reflux for 90 min. Methanol (10 mL) was added and the mixture was heated under reflux for 1 h. The solvent was removed and the residue was loaded onto an SCX2 cartridge with methanol. Washing with methanol followed by elution with 0.7M ammonia in methanol gave the title compound as a viscous oil containing solvent. MS $[M+H]^+$ (ES+) 360/362

The following intermediates were prepared analogously from the appropriate aryloxy piperidine as a mixture of isomers:

| Intermediate | Name | M+H | $^1H$ NMR |
|---|---|---|---|
| 10 | 4-[[4-(2,4-Dichloro-3-methylphenoxy)-1-piperidinyl]methyl]-1,2-cyclopentanediol | 374/376 | $\delta_{(CD3OD)}$ 1.28 - 1.37 (0.66H, m), 1.37 - 1.48 (1.34H, m), 1.69 - 1.81 (4H, m), 1.84 - 2.09 (3H, m), 2.27 (2H, d), 2.35 (3H, s), 2.36 - 2.53 (2H, m), 2.61 - 2.76 (2H, m), 3.78 - 3.85 (0.66H, m), 3.90 - 3.96 (1.34H, m), 4.32 - 4.43 (1H, m), 6.85 (1H, d), 7.16 (1H, d) |
| 11 | 4-[[4-(3,4-Dichloro-2-methylphenoxy)-1-piperidinyl]methyl]-1,2-cyclopentanediol | 374/376 | $\delta_{(CD3OD)}$ 1.28 - 1.36 (0.66H, m), 1.39 - 1.48 (1.34H, m), 1.68 - 1.80 (4H, m), 1.86 - 1.98 (3H, m), 2.22 (3H, s), 2.25 (2H, d), 2.29 - 2.50 (2H, m), 2.60 - 2.70 (2H, m), 3.78 - 3.84 (0.66H, m), 3.89 - 3.95 (1.34H, m), 4.29 - 4.38 (1H, m), 6.82 (1H, d), 7.18 (1H, d) |

INTERMEDIATE 12

4-(3,4-Dichlorophenoxy)-1-(4-piperidinylmethyl)-piperidineacetonitrile

4-(3,4-Dichlorophenoxy)-1-(4-piperidinylmethyl)-piperidine (0.5 g), bromoacetonitrile (0.21 g), diisopropylethylamine (0.36 mL) and dimethylformamide (3 mL) were stirred together at room temperature, under nitro gen, for 4 hours. The mixture was poured into water (50 mL), extracted into ethyl acetate (3 x 50 mL), washed with brine (50 mL), dried, filtered and evaporated. Flash chromatography (29:1 dichloromethane: methanol) gave the title compound as a solid (363 mg).

MS $[M+H]^+$ (APCI+) 382/384

$^1H$ NMR $\delta_{(CDCL3)}$ 1.24 (2H, qd), 1.45 - 1.55 (1H, m), 1.73 - 1.85 (4H, m), 1.92-2.00 (2H, m), 2.19 (2H, d), 2.20 - 2.27 (2H, m), 2.34 (2H, td), 2.63 - 2.71 (2H, m), 2.80 (2H, d), 3.53 (2H, s), 4.21 - 4.28 (1H, m), 6.75 (1H, dd), 6.99 (1H, d), 7.30 (1H, d)

The following intermediates were prepared analogously from the appropriate aryloxy piperidine:

| Intermediate | Name | M+H | $^1$HNMR |
|---|---|---|---|
| 13 | 4-[[4-(3,4-Dichloro-2-methylphenoxy)-1-piperidinyl]methyl]-1-piperidineacetonitrile | 396/398 | |

INTERMEDIATE 14

4-[[4-(3,4-Dichlorophenoxy)-1-piperidinyl]methyl]-1-piperidinepropanenitrile

4-(3,4-Dichlorophenoxy)-1-(4-piperidinylmethyl)-piperidine (0.85 g), acrylonitrile (0.24 mL), diisopropylethylamine (0.72 mL) and dimethylformamide (6 mL) were stirred together at room temperature, under nitrogen, for 24 hours. The mixture was poured into water (50 mL), extracted into ethyl acetate (3 x 50 mL), washed with brine (50 mL), dried, filtered and evaporated. Flash chromatography (19:1 dichloromethane: methanol) gave the title compound as a solid (116 mg).

MS $[M+H]^+$ (APCI+) 396/398

$^1H$ NMR $\delta_{(CD3OD)}$ 1.06 - 1.23 (2H, m), 1.40 - 1.53 (1H, m), 1.60 - 1.75 (4H, m), 1.84 - 1.93 (2H, m), 1.95 - 2.06 (2H, m), 2.11 - 2.17 (2H, m), 2.17 - 2.30 (2H, m), 2.43 - 2.70 (6H, m), 2.76 - 2.95 (2H, m), 4.20 - 4.40 (1H, m), 6.78 (1H, dd), 6.99 (1H, d), 7.28 (1H, d)

INTERMEDIATE 15

4-[[4-(3,4-Dichlorophenoxy)-1-piperidinyl]methyl]-1-piperidineethanamine

4-[[4-(3,4-Dichlorophenoxy)-1-piperidinyl]methyl]-1piperidineacetonitrile (0.43 g) and cobalt (II) chloride (0.3 g) in methanol (20 mL) were cooled to 5 °C, under nitrogen, and sodium borohydride (0.43 g) was added portionwise. The

mixture was stirred at 5 °C for 40 minutes then poured into 2N aqueous sodium hydroxide solution (50 mL), extracted into ethyl acetate (3 x 50 mL), dried, filtered and evaporated to give the title compound (0.43 g).
$^1$H NMR $\delta_{(CDCl3)}$ 1.08 - 1.28 (3H, m), 1.50 - 1.80 (6H, m), 1.88 - 2.02 (3H, m), 2.04 - 2.22 (4H, m), 2.45 (1H, s), 2.56 - 2.73 (3H, m), 2.89 (2H, m), 3.07 - 3.10 (1H, d), 4.23 (1H, m), 6.74 - 6.76 (1H, d), 6.99 (1H, s), 7.26 -7.31 (1H, t)
The following intermediates were prepared analogously from the appropriate nitrile:

| Intermediate | Name | M+H | $^1$H NMR |
|---|---|---|---|
| 16 | 4-[[4-(3,4-Dichloro-2-methylphenoxy)-1-piperidinyl]methyl]-1-piperidineethanamine | | $\delta$($_{CDCl3}$) 1.09 - 1.26 (3H, m), 1.62 - 1.85 (6H, m), 1.88 - 2.01 (3H, m), 2.16-2.18 (2H, d), 2.21- 2.30 (2H, m), 2.32 (3H, s), 2.39 - 2.44 (1H, m), 2.58 -2.71 (3H, m), 2.75 -2.98 (2H, t), 3.03 - 3.16 (1H,d), 4.62 (1H,m), 6.70 -6.73 (1H, d), 7.19-7.22 (1H, d) |
| 17 | 4-[[4-(3,4-Dichlorophenoxy)-1-piperidinyl] methyl]-1-piperidinepropanamine | | $\delta_{(CDCl3)}$ 1.02-1.23 (2H, m), 1.40 - 1.83 (11H, m), 1.85- 1.94 (3H, m), 2.08- 2.20 (4H, m), 2.65 (3H, m), 2.93 -3.20 (2H, m), 4.23 (1H, m), 6.73- 6.76 (1H, d), 6.99 (1H, s), 7.26-7.31 (1H, t) |

INTERMEDIATE 18

1-Methylethyl 3-formyl-2-pyridinecarboxylate

1-Methylethyl 3-(hydroxymethyl)-2-pyridinecarboxylate (1.2 g) was dissolved in dichloromethane (20 mL) and to the solution was added Dess-Martin periodinane (3.0 g). The reaction mixture was stirred, under nitrogen, at room temperature, for 1 h. Sodium thiosulphate (10 g) was added to a saturated aqueous solution of sodium bicarbonate (25 mL) and this mixture was added to the reaction mixture. Ether (25 mL) was then added and the mixture was stirred rapidly for 5min. The mixture was separated, the aqueous phase was extracted with ether (2x20 mL). 2M HCl (10 mL) was added to the combined ether extracts. The aqueous phase was removed, basified by careful addition of solid sodium bicarbonate and extracted with ether. This ether was dried ($MgSO_4$), filtered and concentrated *in vacuo* to give the title compound as a colourless oil (0.87 g).
$^1$H NMR $\delta_{(DMSO)}$ 1.35 (6H, d), 5.24 (1H, quintet), 7.80 (1H, dd), 8.31 (1H, dd), 8.86 (1H, dd), 10.29 (1H, s)

INTERMEDIATE 19

Methyl 4-(bromomethyl)-3-fluoro- benzoate

Methyl 3-fluoro-4-methyl benzoate (0.97 g), N-bromosuccinimide (1.13 g) and azobisisobutyronitrile (0.02 g) were added to carbon tetrachloride (2 mL) and the mixture was heated under reflux, whilst being irradiated with a 100W lamp, for 6h. The reaction mixture was concentrated *in vacuo* and the residue was partitioned between ethyl acetate and 1 M hydrochloric acid. The organic phase was washed with brine, dried ($MgSO_4$) and filtered to give a crude yellow oil which was purified by flash chromatography, eluting with 5% ethyl acetate in isohexane to give the title compound as a colourless oil (0.63 g). $^1$H NMR $\delta_{(CDCl3)}$ 3.93 (3H, s), 4.52 (2H, d), 7.47 (1H, t), 7.73 (1H, dd), 7.81 (1H, dd)

INTERMEDIATE 20

Methyl 2-(bromomethyl)-5-fluoro benzoate

Prepared following the method for Intermediate 15.
$^1$H NMR $\delta_{(CDCl3)}$ 3.95 (3H, s), 4.93 (2H, s), 7.20 (1H, ddd), 7.46 (1H, dd), 7.67 (1H, dd)

INTERMEDIATE 21

Ethyl 4-[(4-hydroxy-1-piperidinyl)methyl]-α-phenyl-1-piperidineacetate

a) Ethyl4-(hydroxymethyl)-α-phenyl-1-piperidineacetate

Ethyl α-bromobenzeneacetate (2.43 g) was dissolved in acetone (20 mL). A suspension of 4-hydroxymethylpiperidine (1.15 g) in acetone (5 mL) was added followed by potassium carbonate (2.60 g). The mixture was stirred for 16 h, filtered and concentrated to an oil. Chromatography (isohexane : ethyl acetate 1:1, then 3:7) gave the subtitle compound as an oil (2.23 g).
MS $[M+H]^+$ (ES+) 278
$^1$H NMR $\delta_{(CDCl3)}$ 1.21 (3H, t), 1.32 (1H, td), 1.41 (1H, td), 1.46 - 1.57 (1H, m), 1.63 - 1.69 (1H, m), 1.70- 1.77 (1H, m), 1.89 (1H, td), 2.16 (1H, td), 2.76-2.81 (1H, m), 2.98-3.04 (1H, m), 3.50 (2H, d), 3.99 (1H, s), 4.09 - 4.24 (2H, m), 7.30 - 7.37 (3H, m), 7.42 - 7.46 (2H, m)

b) Ethyl 4-formyl-α-phenyl-1-piperidineacetate

DMSO (1.1 mL) was dissolved in dichloromethane (15 mL) and cooled below - 60°C. Oxalyl chloride (0.9 mL) in dichloromethane (5 mL) was added dropwise maintining the temperature below -57°C. The solution was stirred for 15 min. then ethyl 4-(hydroxymethyl)-α-phenyl-1-piperidineacetate (2.23 g) dissolved in dichloromethane (6 mL) was added dropwise and the solution was stirred for 30 min. Triethylamine (4 mL) was added and the reaction mixture was allowed to warm to ambient temperature. Water was added, the phases were separated, the aqueous was extracted twice with dichloromethane and the organic phases were washed with brine, dried, filtered and concentrated to give the subtitle compound.
MS $[M+H]^+$ (ES+) 276
$^1$H NMR $\delta_{(CDCl3)}$ 1.21 (3H, t), 1.64 - 1.81 (2H, m), 1.82 - 1.95 (1H, m), 2.11 (1H, td), 2.19 - 2.34 (2H, m), 2.70 - 2.80 (2H, m), 2.81 - 2.90 (1H, m), 4.04 (1H, s), 4.07 - 4.25 (2H, m), 7.30 - 7.38 (3H, m), 7.39 - 7.44 (2H, m), 9.63 (1H, d)

c) Ethyl 4-[(4-hydroxy-1-piperidinyl)methyl]-α-phenyl-1-piperidineacetate

4-Hydroxypiperidine (0.81 g) and ethyl 4-formyl-α-phenyl-1-piperidineacetate (2.14 g) were suspended in THF (10 mL). Acetic acid (0.5 mL) was added followed by sodium triacetoxyborohydride (1.68 g) and then THF (6 mL). The suspension was stirred overnight, then sodium bicarbonate solution was added and the mixture was stirred for 5 min. The suspension was extracted thrice with ethyl acetate, the organic phases were washed with brine, dried, filtered and evaporated. Chromatography of the residue (dichloromethane : methanol: triethylamine 90:9:1) gave the subtitle compound as an oil (2.14 g).
MS $[M+H]^+$ (ES+) 361
$^1$H NMR $\delta_{(CDCL3)}$ 1.20 (3H, td), 1.33 (2H, qd), 1.42 - 1.49 (1H, m), 1.49 - 1.57 (2H, m), 1.69 - 1.76 (2H, m), 1.81 - 1.89 (3H, m), 2.00 - 2.12 (3H, m), 2.14 (2H, d), 2.58 - 2.78 (4H, m), 2.93 - 2.98 (1H, m), 3.61 - 3.70 (1H, m), 3.97 (1H, s), 4.07 - 4.23 (2H, m), 7.29 - 7.36 (3H, m), 7.41 - 7.45 (2H, m).

EXAMPLE 1

This Example illustrates the preparation of 4-[[4-(3,4-dichlorophenoxy)-1-piperidinyl]methyl]-α-phenyl- 1-piperidineacetic acid.

4-{[4-(3,4-Dichlorophenoxy)piperidin-1-yl]methyl}piperidine (0.5 g), and benzene boronic acid (0.2 g) were dissolved in ethanol (3 mL); glyoxylic acid (0.2 mL of a 50% solution in water) was added to the solution and the reaction mixture was heated in a microwave oven at 100°C for 5min. The resultant solution was purified by HPLC (gradient 95% - 5% aqueous ammonium acetate, 5% - 95% acetonitrile) to give the title compound (0.1 g).
MS $[M+H]^+$ (ES+) 477/479
$^1$H NMR δ ($CDCl_3$) 1.53 - 1.77 (4H, m), 1.79 - 1.94 (4H, m), 2.14 - 2.25 (4H, m), 2.41 (1H, t), 2.54 - 2.64 (2H, m), 2.75 (1H, t), 3.38 (1H, d), 3.58 - 3.70 (2H, m), 4.15 - 4.23 (1H, m), 4.47 (1H, s), 6.71 (1H, dd), 6.96 (1H, d), 7.25 (1H, d), 7.32 - 7.38 (3H, m), 7.49 - 7.58 (2H, m).

Examples 2-19 (see Table I below) were made using the method of Example 1.

EXAMPLE 20

This Example illustrates the preparation of 4-[[4-(2,5-dichlorophenoxy)-1-piperidinyl]methyl]-α-phenyl-1-piperidineacetic acid

Ethyl 4-[(4-hydroxy-1-piperidinyl)methyl]-α-phenyl-1-piperidineacetate (0.135 g) was dissolved in NMP (3 mL). 1,4-Dichloro-2-fluorobenzene (0.2 mL) and potassium t-butoxide (56 mg) were added and the solution was heated to 50 °C for 40 h. The solution was cooled to ambient temperature and few drops of aqueous sodium hydroxide solution were added. The mixture was stirred for 60 h, then acetic acid (few drops) was added and the solvent was distilled. The residue was purified by HPLC (0.2% aqueous ammonia : acetonitrile; gradient 95:5 to 50:50) to give the title compound (21 mg).

MS $[M+H]^+$ (ES+) 477/479

$^1H$ NMR $\delta_{(CD3OD)}$ 1.45 (1H, q), 1.68 - 1.96 (9H, m), 2.16 - 2.21 (2H, m), 2.25 - 2.34 (2H, m), 2.57 - 2.65 (3H, m), 2.80 - 2.93 (2H, m), 4.29 - 4.36 (1H, m), 4.38 - 4.44 (1H, m), 6.83 (1H, dd), 7.02 (1H, d), 7.23 (1H, d), 7.32 - 7.36 (3H, m), 7.44 - 7.49 (2H, m)

Example 21 (see Table I below) was made using the method of Example 20

EXAMPLE 22

This Example illustrates the preparation of methyl 4-[[4-(3,4-dichlorophenoxy)-1-piperidinyl]methyl]-α-phenyl-piperidineacetate.

4-(3,4-Dichlorophenoxy)-1-(4-piperidinylmethyl)-piperidine (0.30 g) and methyl-α-bromobenzeneacetate (0.22 g) were dissolved in acetone (20 mL) and potassium carbonate (0.13 g) was added. The reaction mixture was stirred at room temperature for 16 h. The suspension was filtered and the filtrate was evaporated. The residue was chromatographed eluting with ethyl acetate : methanol : triethylamine (20 : 1 : 0.001) to give the title compound (0.24 g).

MS $[M+H]^+$ (ES+) 491 /493

$^1H$ NMR $\delta_{(CD3OD)}$ 1.22 (1H, qd), 1.34 (2H, qd), 1.50 - 1.59 (1H, m), 1.66 (1H, d), 1.70 - 1.80 (3H, m), 1.88 (1H, td), 1.93 - 2.02 (2H, m), 2.14 (1H, td), 2.22 (2H, d), 2.25 - 2.33 (1H, m), 2.65 - 2.73 (3H, m), 2.95 - 3.01 (1H, m), 3.68 (3H, s), 3.98 (1H, s), 4.37 (1H, septet), 6.87 (1H, dd), 7.08 (1H, d), 7.31 - 7.38 (4H, m), 7.42 (2H, dd)

EXAMPLES 23 & 24

This Example illustrates the preparation of (*R*)-methyl 4-[[4-(3,4-dichlorophenoxy)-1-piperidinyl]methyl]-α-phenyl-piperidineacetate and (*S*)-methyl 4-[[4-(3,4-dichlorophenoxy)-1-piperidinyl]-methyl]-α-phenyl-piperidineacetate.

Racemic methyl 4-[[4-(3,4-dichlorophenoxy)-1-piperidinyl]methyl]-α-phenylpiperidineacetate (360 mg) was dissolved in isohexane : isopropanol (9:1) and was chromatographed on a Chiralpak AD column eluting isohexane : isopropanol (9:1) to give the 2 isomers.

First eluting isomer (50 mg); MS $[M+H]^+$ (ES+) 491/493. Retention time (chiralpak AD column (4.6 x 250 mm), maintained at 10 °C, flow rate 1 mL/min 95:5 isohexane : isopropanol containing 0.1% diethylamine) 7.2 minutes.

Second eluting isomer (30 mg); MS $[M+H]^+$ (ES+) 491/493. Retention time (chiralpak AD column (4.6 x 250 mm), maintained at 10 °C, flow rate 1 mL/min 95:5 isohexane : isopropanol containing 0.1 % diethylamine) 8.9 minutes

EXAMPLE 25

This Example illustrates the preparation of (*R*)- 4-[[4-(3,4-dichlorophenoxy)-1-piperidinyl]methyl]-α-phenyl-piperidineacetic acid.

Methyl (*R*)-4-[[4-(3,4-dichlorophenoxy)-1-piperidinyl]methyl]-α-phenyl-1-piperidineacetate (45 mg) was dissolved in aq. HCl (6M, 10 mL) and heated at 80° C for 22hrs. It was dried on a rotary evaporator, redissolved in MeOH and purified by HPLC (gradient 95% - 50% aqueous ammonium acetate, 5% - 50% acetonitrile) to give the title compound (14.1 mg).

MS $[M+H]^+$ (ES+) 477/479

$^1H$ NMR $\delta_{(CD3OD + NaOD)}$ 1.27 - 1.37 (2H, m), 1.45 - 1.62 (2H, m), 1.72 - 2.06 (8H, m), 2.31 - 2.36 (2H, m), 2.36 - 2.45 (2H, m), 2.72 - 2.80 (2H, m), 2.97 (1H, t), 4.37 - 4.46 (2H, m), 6.88 (1H, dd), 7.09 (1H, d), 7.37 (1H, d), 7.42 - 7.46 (3H, m), 7.54 - 7.58 (2H, m)

Example 26 (see Table I below) was made using the method of Example 25

EXAMPLE 27

This Example illustrates the preparation of (*R*)-methyl 4-[[4-(3,4-dichlorophenoxy)-1-piperidinyl]methyl]-α-phenyl-piperidineacetate

4-[[4-(3,4-Dichlorophenoxy)-1-piperidinyl]methyl]-1,2-cyclopentanediol (Intermediate 9, major isomer, 230 mg) was dissolved in dichloromethane (5 mL). Sodium carbonate (225 mg) was added and the resulting suspension was

cooled in ice-water. Lead tetraacetate (310 mg) was added in small portions over 15 min. A suspension of (*R*)-phenylglycine methyl ester hydrochloride (129 mg) and sodium triacetoxyborohydride (300 mg) in THF (10 mL) was prepared in a separate flask. To this suspension was added acetic acid (50 μL) and triethylamine (100 μL) then the suspension was sonicated (cleaning bath) for 5 min. 40 min after the completion of the addition of lead tetraacetate to the diol the resulting suspension was filtered through a plug of cotton wool into the aminoester suspension, followed by a rinse of THF (3 mL). Additional acetic acid (50 μL) and triethylamine (100 μL) were added to the reaction mixture which was then stirred overnight.

Aqueous sodium bicarbonate was added to the reaction mixture which was then extracted thrice with ethyl acetate. The extracts were combined, washed with brine, dried, filtered and evaporated. The residue was purified by chromatography (39:1 ethyl acetate : methanol) to give the title compound (157 mg).
MS $[M+H]^+$ (ES+) 491/493
$^1H$ NMR $\delta_{(CDCl3)}$ 1.24 (1H, qd), 1.33 (1H, qd), 1.41 - 1.52 (1H, m), 1.70 - 1.80 (3H, m), 1.85 (1H, td), 1.90 - 1.98 (2H, m), 2.12 (1H, td), 2.16 - 2.25 (5H, m), 2.62 - 2.69 (2H, m), 2.75 (1H, d), 2.94 (1H, d), 3.69 (3 H, s), 4.01 (1H, s), 4.19 - 4.26 (1H, m), 6.74 (1H, dd), 6.98 (1H, d), 7.29 (1H, d), 7.31 - 7.36 (3H, m), 7.40 - 7.44 (2H, m)

Examples 28 - 33 (see Table I below) were made using the method of Example 27

<u>EXAMPLE 34</u>

This Example illustrates the preparation of (*R*)- 4-[[4-(3,4-dichlorophenoxy)-1-piperidinyl]methyl]-α-phenyl-piperidineacetic acid dihydrochloride

(*R*)-Methyl 4-[[4-(3,4-dichlorophenoxy)-1-piperidinyl]methyl]-α-phenylpiperidineacetate (150 mg) was suspended in 6M hydrochloric acid (20 mL) and heated to 80 °C for 22 h. The crystalline solid formed was collected and dried *in vacuo* to give the title compound (100 mg).
m. pt. 294-297 C
MS $[M+H]^+$ (ES+) 477/479 ppp
$^1H$ NMR $\delta_{(CD3OD)}$ 1.43 - 1.59 (1H, m), 1.66 (1H, q), 1.86 - 2.02 (2H, m), 2.05 - 2.29 (5H, m), 2.78 - 2.93 (1H, m), 2.98 - 3.18 (12H, m), 3.37 - 3.45 (2H, m), 3.61 (1H, d), 3.74 - 3.88 (1H, m), 4.47 - 4.57 (0H, m), 4.67 - 4.72 (1H, m), 5.00 - 5.12 (1H, m), 6.83 - 6.91 (1H, m), 7.09 - 7.16 (1H, m), 7.31 - 7.36 (1H, m)

Examples 35 - 40 (see Table I below) were made using the method of Example 25.

<u>EXAMPLE 41</u>

This Example illustrates the preparation of 1-[4-[[4-(3,4-dichlorophenoxy)-1-piperidinyl]methyl]-1-piperidinyl]-2,3-dihydro-1*H*-indene-1-carboxylic acid

4-[[4-(3,4-Dichlorophenoxy)-1-piperidinyl]methyl]-1,2-cyclopentanediol (0.20 g) was dissolved in dichloromethane (10 mL) and sodium carbonate (0.206 g) was added. The suspension was cooled to 0°C. Lead tetraacetate (0.248 g) was added over 20 minutes. The mixture was stirred for 40 min at 0 C.

The suspension was filtered through a plug of cotton wool into a solution of 1-amino-2,3-dihydro-1*H*-indene-1-carboxylic acid (0.098 g), hydrochloric acid (0.1 mL), triethylamine (0. 1 mL) and methanol (10 mL). Sodium cyanoborohydride (0.052 g) was added and the reaction mixture was stirred for 16 h at room temperature. The solvents were evaporated and the residue was redissolved in acetonitrile/water and AcOH was added. This was purified by HPLC (5% MeCN/95%$NH_4$0Ac aq (0.1%) gradient to 50% MeCN/50%$NH_4$OAc) to give title compound (93 mg).
MS $[M+H]^+$ (ES+) 503/505.
$^1H$ NNM $\delta_{(CD3OD + NaOD)}$ 1.17 - 1.27 (1H, m), 1.29 - 1.41 (2H, m), 1.46 - 1.54 (1H, m), 1.54 - 1.70 (3H, m), 1.83 - 1.93 (3H, m), 1.97 - 2.24 (6H, m), 2.42 - 2.52 (2H, m), 2.55 - 2.65 (2H, m), 2.71 - 2.80 (1H, m), 2.87 - 3.05 (2H, m), 4.22 - 4.31 (1H, m), 6.74 - 6.80 (1H, m), 6.97 - 7.03 (4H, m), 7.27 (1H, d), 7.44 (1H, d)

<u>EXAMPLE 42</u>

This Example illustrates the preparation of methyl 2-[(4-{[4-(3,4-dichlorophenoxy)piperidin-1-yl]methyl} piperidin-1 -yl)methyl]benzoate

4-(3,4-Dichlorophenoxy)-1-(piperidin-4-ylmethyl)piperidine (0.5 g) was dissolved in acetonitrile (2 mL) and to the solution was added methyl 2-(bromomethyl)benzoate (0.56 g) and DIPEA (0.25 mL). The reaction mixture was stirred at room temperature overnight then concentrated by evaporation under reduced pressure. The residue was partitioned between ethyl acetate and water, the organic phase was washed with brine, dried ($MgSO_4$), filtered and concentrated to give an oil. This was purified by chromatography eluting with 5% methanol in dichloromethane then by HPLC (25% MeCN/75%$NH_4$OAc aq (0.1 %) gradient to 95% MeCN/5%$NH_4$OAc) to give the title compound as an oil 0.4 g.
MS $[M+H]^+$ (ES+) 491/493.

$^1H$ NMR $\delta_{(CDCl3)}$ 1.10 - 1.24 (2H, m), 1.46 (1H, qd), 1.63 - 2.05 (8H, m), 2.15 - 2.28 (4H, m), 2.62 - 2.71 (2H, m), 2.76 - 2.82 (2H, m), 3.74 (2H, s), 3.87 (3H, s), 4.23 (1H, quintet), 6.74 (1H, dd), 6.99 (1H, d), 7.25 - 7.32 (2H, m), 7.37 - 7.46 (2H, m), 7.68 (1H, d).

Example 43 (see Table I below) were made using the method of Example 42.

EXAMPLE 44

This Example illustrates the preparation of methyl 2-[[4-[[4-(2,4-dichlorophenoxy)-1-piperidinyl]methyl]-1-piperidinyl]methyl]-5-fluoro-benzoate

4-(2,4-Dichlorophenoxy)-1-(4-piperidinylmethyl)-piperidine dihydrochloride (0.26 g) was added to acetonitrile (3 mL) and treated with triethylamine (0.26 mL). After stirring for 5min, methyl 2-(bromomethyl)-5-fluoro benzoate (0.15 g) was added and the reaction mixture was stirred at room temperature overnight. The reaction mixture was concentrated *in vacuo* and crude product was purified by flash chromatography, eluting with 2% methanol and 0.1 % triethylamine in dichloromethane, giving the title compound contaminated with triethylamine hydrochloride.

MS $[M+H]^+$ (ES+) 509/511

Examples 45, 48-50 were prepared following the method of example 44.

EXAMPLE 46

This Example illustrates the preparation of 1-methylethyl- 3-[[4-[[4-(3,4-dichlorophenoxy)-1-piperidinyl]methyl]-1-piperidinyl]methyl]-2-pyridinecarboxylate

4-(3,4-Dichlorophenoxy)-1-(4-piperidinylmethyl)-piperidine (0.3 g) and 1-methylethyl-3-formyl-2-pyridinecarboxylate (0.17 g) were added to a mixture of THF (3 mL) and acetic acid (0.5 mL). The mixture was stirred at room temperature for 5min then sodium triacetoxyborohydride (0.28 g) was added. The mixture was stirred overnight then poured into a saturated solution of sodium bicarbonate. The product was extracted with ethyl acetate, the organic phase was washed with brine, dried ($MgSO_4$), filtered and concentrated *in vacuo.* The residue was purified by flash chromatography, eluting with 3% methanol and 0.1 % triethylamine in dichloromethane, giving the title compound as a clear oil (0.24 g).

$^1H$ NMR $\delta_{(CD3OD)}$ 1.13 - 1.28 (2H, m), 1.43 (6H, d), 1.50 - 1.65 (1H, m), 1.69 - 1.83 (4H, m), 1.96 - 2.11 (4H, m), 2.23 (2H, d), 2.27 - 2.37 (2H, m), 2.67 - 2.84 (4H, m), 3.72 (2H, s), 4.35 - 4.45 (1H, m), 5.26 (1H, t), 6.90 (1H, dd), 7.11 (1H, d), 7.39 (1H, d), 7.52 (1H, dd), 7.93 (1H, dd), 8.49 (1H, dd)

Examples 47, 60 - 66 (Table I below) were prepared following the method of Example 46.

EXAMPLES 51-59

Examples 51-59 (Table I below) were made from Examples 42-50 by the methods of Example 77 (LiOH, Examples 51, 53, 54, 57, 58, 59), Example 25 (HCl, Examples 55, 56) or Example 90 (KOTMS, Example 52).

EXAMPLE 67

This Example illustrates the preparation of methyl 4-[[4-(3,4-dichlorophenoxy)-1-piperidinyl]methyl]-1-piperidineacetate

To a stirred solution of 4-(3,4-dichlorophenoxy)-1-(4-piperidinylmethyl)-piperidine (0.23 g) and DIPEA (0.164 mL) in DMF at RT was added methyl bromoacetate (0.076 mL). The reaction was heated at 60°C for 16 h. Saturated sodium bicarbonate solution (30 mL) was then added to the cooled solution and the product was extracted into ethyl acetate (3 x 20 mL). The combined organics were washed with brine (10 mL) and then dried, filtered and evaporated to leave a colourless oil (0.135 g).

MS $[M+H]^+$ (ES+) 415/417

Examples 68-72 (see Table I) were prepared analogously to Example 67 from the appropriate amine.

EXAMPLE 73

This Example illustrates the preparation of methyl (2*R*)-2-(4-{[4-(3,4-dichlorophenoxy)piperidin-1-yl]methyl}piperidin-1-yl)propanoate

Diethyl ether (10 mL) and dimethylformamide (2 mL) were added to 4-(3,4-dichlorophenoxy)-1-(piperidin-4-ylmethyl)piperidine (0.32 g) and the mixture was sonicated (cleaning bath) until it became clear. Methyl (2S)-2-bromopropanoate (0.16 g) and triethylamine (0.6 mL) were added and the mixture was stirred at room temperature overnight. The reaction mixture was poured into water and was extracted with diethyl ether. The diethyl ether was washed with

brine, dried ($MgSO_4$), filtered and concentrated under reduced pressure to give an oil. Crude product was purified by chromatography, eluting with 95 : 5 : 0.1 dichloromethane : methanol : aqueous ammonia to give the title compound as an oil (0.25 g).
MS $[M+H]^+$ (ES+) 429/431
Examples 74-76 (see Table I) were prepared analogously to Example 73.

EXAMPLE 77

This Example illustrates the preparation of 4-[[4-(3,4-dichlorophenoxy)-1-piperidinyl]methyl]-1-piperidineacetic acid.
Methyl 4-[[4-(3,4-dichlorophenoxy)-1-piperidinyl]methyl]-1-piperidineacetate (0.135 g) and lithium hydroxide (0.136 g) in 3 :1 methanol/water (2 mL) was stirred at RT for 16 h. The reaction mixture was acidified to pH 4 with acetic acid and purified by HPLC (10% MeCN/90%$NH_4$OAc aq (0.1%) gradient to 70% MeCN/30%$NH_4$OAc) to provide the title compound as a white solid (0.030 g).
MS $[M+H]^+$ (ES+) 401/403.
$^1$H NMR $\delta_{(CD3OD)}$ 1.52 (2H, qd), 1.72 - 1.92 (3H, m), 1.98 - 2.09 (4H, m), 2.34 (2H, d), 2.38 - 2.45 (2H, m), 2.72 - 2.83 (2H, m), 3.01 (2H, td), 3.56 - 3.67 (4H, m), 4.35 - 4.49 (1H, m), 6.90 (1H, dd), 7.11 (1H, d), 7.39 (1H, d).
Examples 78-86 (see Table I) were prepared analogously to Example 77 from the appropriate ester.

EXAMPLE 87

This Example illustrates the preparation of methyl 4-[[4-(3,4-dichlorophenoxy)-1-piperidinyl]methyl]-α,α-dimethyl-1-piperidine propanoate
To a stirred solution of 4-{[4-(3,4-dichlorophenoxy)piperidin-1-yl]methyl}piperidine (0.175 g) and 2,2-dimethyl-3-oxopropanoic acid methyl ester (80 mg) in THF (0.5 mL) was added sodium triacetoxyborohydride (162 mg) and acetic acid (0.041 mL). The reaction mixture was stirred at room temperature overnight. Saturated sodium bicarbonate solution (30 mL) was added and the product was extracted into ethyl acetate (3 x 20 mL). The combined organics were washed with brine (10 mL) and dried ($MgSO_4$), filtered and evaporated to leave an oil (0.17 g). A portion (0.080 g) was purified by HPLC (5% MeCN/95%$NH_4$OAc aq (0.1 %) gradient to 5% MeCN/95%$NH_4$OAc) to give the title compound as an oil (0.012 g).
MS $[M+H]^+$ (ES+) 457/459.
$^1$H NMR $\delta_{(CDCl3)}$ 1.15 (6H, s), 1.16 (1H, qd), 1.34 - 1.45 (1H, m), 1.58 - 1.62 (2H, m), 1.62- 1.66 (2H, m), 1.71 - 1.82 (2H, m), 1.90 - 2.00 (2H, m), 2.07 - 2.16 (3H, m), 2.16 - 2.26 (2H, m), 2.45 (2H, s), 2.60 - 2.70 (2H, m), 2.70 - 2.77 (2H, m), 3.65 (3H, s), 4.18 - 4.27 (1H, m), 6.74 (1H, dd), 6.99 (1H. d), 7.30 (1H, d).
Examples 88 & 89 (see Table I) were prepared analogously to Example 87 from the appropriate amines.

EXAMPLE 90

This Example illustrates the preparation of 4-[[4-(3,4-dichlorophenoxy)-1-piperidinyl]methyl]-α,α-dimethyl-1-piperidine propanoic acid.
To a stirred solution of methyl 4-[[4-(3,4-dichlorophenoxy)-1-piperidinyl]methyl]-α,α-dimethyl-1-piperidine propanoate (0.080 g) in THF (1 mL) at RT was added potassium trimethylsilanolate (27 mg). After 16 h the reaction mixture was incomplete and further potassium trimethylsilanolate (27 mg) was added. After a further 1 h the reaction solvent was evaporated and the residue was redissolved in acetonitrile and purified by HPLC (5% MeCN/95%$NH_4$OAc aq (0.1 %) gradient to 60% MeCN/40%$NH_4$OAc) to give the title compound (0.036 g).
MS $[M+H]^+$ (ES+) 443/445.
$^1$H NMR $\delta_{(CD30D)}$ 1.22 (6H, s), 1.47 (2H, q), 1.68 - 1.81 (2H, m), 1.79 - 1.88 (1H, m), 1.93 - 2.05 (4H, m), 2.27 (2H, d), 2.33 (2H, t), 2.67 - 2.76 (2H, m), 2.95 - 3.02 (2H, m), 3.04 (2H, s), 3.45 - 3.52 (2H, m), 4.33 - 4.42 (1H, m), 6.87 (1H, dd), 7.08 (1H, d), 7.36 (1H, d).
Example 91 & 92 (Table I) were prepared analogously to Example 90 from the appropriate esters

EXAMPLE 93

This Example illustrates the preparation of 4-[[4-(3,4-Dichlorophenoxy)-1-piperidinyl]methyl]-1-piperidine propanoic acid dihydrochloride
To a stirred solution of 4- {[4-(3,4-dichlorophenoxy)piperidin-1-yl]methyl }piperidine (0.175 g) in isopropanol (0.4 mL) at RT was added acrylic acid (0.038 mL). After 16 h the reaction mixture was purified by HPLC (5% MeCN/95%$NH_4$OAc aq (0.1 %) gradient to 50% MeCN/50%$NH_4$OAc). Treatment of the product with 2 M HCl at 40 °C for 15

min followed by evaporation left a yellow solid. This was triturated with diethyl ether (3 mL) and the residual solid was partially dissolved in 4 : 1 dichloromethane/methanol. The supernatant was evaporated to provide the title compound as a solid (0.014 g).
MS $[M+H]^+$ (ES+) 415/417.
$^1H$ NMR $\delta_{(D2O)}$ 1.63 (2H, qd), 1.91 - 2.05 (1H, m), 2.09 - 2.21 (2H, m), 2.26 (2H, d), 2.29 - 2.36 (1H, m), 2.40 (1H, d), 2.87 (2H, t), 3.08 (2H, t), 3.14 - 3.22 (2H, m), 3.29 - 3.40 (2H, m), 3.44 (2H, t), 3.52 (1H, d), 3.64 - 3.79 (3H, m), 4.61 - 4.70 (1H, m), 6.96 - 7.03 (1H, m), 7.24 - 7.29 (1H, m), 7.50 (1H, d).

EXAMPLE 94

This Example illustrates the preparation of 4-[[4-(3,4-dichlorophenoxy)-1-piperidinyl]methyl]-1-piperidinebutanoic acid.

4-(3,4-Dichlorophenoxy)-1-(4-piperidinylmethyl)-piperidine (0.20 g) and methyl 4-bromo-butanoate (0.10 g) were dissolved in acetone (20 mL) and potassium carbonate (0.08 g) was added. The reaction mixture was stirred for 16 h at room temperature. The reaction mixture was filtered and the solvents were evaporated to give the title compound (18 mg).
MS $[M+H]^+$ (ES+) 443/445

Example 95 & 96 (Table I) wre prepared analogously to Example 94 from the appropriate halo esters.

Examples 97-99 (Table I) were prepared from the appropriate esters by the method of Example 25.

EXAMPLE 100

This Example illustrates the preparation of 4-(3,4-dichlorophenoxy)-1-[[1-(2H tetrazol-5-ylmethyl)-4-piperidinyl] methyl]-piperidine

4-[[4-(3,4-Dichlorophenoxy)-1-piperidinyl]methyl]-1-piperidineacetonitrile (0.26 g), azidotrimethylsilane (0.5 mL), dibutyltin oxide (0.17 g) and toluene (10 mL) were heated together at 110 °C, in a sealed tube, for 20 hours, cooled and evaporated. The residue was dissolved in methanol, and filtered through reverse-phase silica to remove the tin by-products. The product was further purified by reverse-phase HPLC (25% MeCN/75%$NH_4OAc$ aq (0.1 %) gradient to 95% MeCN/5%$NH_4OAc$). This gave the title compound as a solid (0.24 g).
MS $[M+H]^+$ (APCI+) 425/427.
$^1H$ NMR $\delta_{(CD3OD)}$ 1.16 - 1.38 (2H, m), 1.71 - 1.84 (5H, m), 1.91 - 2.05 (2H, m), 2.37 - 2.49 (2H, m), 2.50 - 2.69 (4H, m), 2.79 - 2.98 (2H, m), 3.20 - 3.25 (2H, m), 4.12 (2H, s), 4.33 - 4.46 (1H, m), 6.81 (1H, dd), 7.04 (1H, d), 7.29 (1H, d)

Example 101 (Table I) was prepared analogously to Example 100 from the appropriate nitrile.

EXAMPLE 102

This Example illustrates the preparation of *N*-[2-[4-[[4-(3,4-dichlorophenoxy)-1-piperidinyl]methyl]-1-piperidi nyl]ethyl]-1,1, 1-trifluoro-methanesulfonamide

4-[[4-(3,4-Dichlorophenoxy)-1-piperidinyl]methyl]-1-piperidineethanamine (0.28 g) in dichloromethane (25 mL) was cooled to - 78°C under nitrogen, and triflic anhydride 0.35 mL) was added dropwise. After 5 minutes the reaction was quenched with excess aqueous ammonia solution, warmed to room temperature, and evaporated. The product was purified by reverse-phase HPLC (25% MeCN/75%$NH_4OAc$ aq (0.1%) gradient to 95% MeCN/5%$NH_4OAc$). This gave the title compound as a solid (0.08 g).
MS $[M+H]^+$ (APCI+) 518/520.
$^1H$ NMR $\delta_{(CD3OD)}$ 1.25 (2H, dd), 1.54 - 1.72 (3H, m), 1.79 (2H, d), 1.85- 1.95 (2H, m), 2.18 (2H, d), 2.25 (2H, t), 2.35 (2H, td), 2.57 - 2.74 (4H, m), 3.11 (2H, d), 3.25 (2H, t), 4.19 - 4.43 (1H, m), 6.79 (1H, dd), 7.00 (1H, d), 7.28 (1H, d)

Examples 103 and 104 (Table I) were prepared analogously to Example 102 from the appropriate amines.

TABLE I

| Example | Name | M+H | $^1$H NMR |
|---|---|---|---|
| 2 | 4-[[4-(2,4-Dichloro-3-methylphenoxy)-1-piperidinyl]methyl]-α-phenyl-1-piperidineacetic acid | 491/493 | $\delta_{(CDCl3)}$ 1.56 - 1.72 (3H, m), 1.83 - 1.97 (6H, m), 2.23 (2H, d), 2.30 - 2.39 (2H, m), 2.45 (3H, s), 2.50 - 2.52 (1 H, m), 2.62 - 2.68 (2H, m), 2.76 - 2.84 (1H, m), 3.39 (1H, d), 3.71 (2H, d), 4.32 (1H, s), 4.57 (1H, s), 6.71 (1H, d), 7.17 (1H, d), 7.36 - 7.38 (3H, m), 7.53 - 7.56 (2H, m) |
| 3 | 4-[[4-(3,4-Dichlorophenoxy)-1-piperidinyl]methyl]-α-(4-fluorophenyl)-1-piperidineacetic acid | 495/497 | $\delta_{(CDCl3)}$ 1.56 - 1.78 (5H, m), 1.84 - 1.98 (4H, m), 2.18 - 2.32 (4H, m), 2.37 - 2.53 (1H, m), 2.57 - 2.67 (2H, m), 2.72 - 2.84 (1H, m), 3.36 - 3.43 (1 H, m), 3.64 - 3.72 (1H, m), 4.19 - 4.26 (1H, m), 4.54 (1H, s), 6.72 (1H, dd), 6.96 (1H, d), 7.07 (2H, t), 7.24 - 7.32 (1 H, m), 7.55 (2H, dd) |
| 4 | 4-[[4-(3,4-Dichlorophenoxy)-1-piperidinyl]methyl]-α-(2-methoxyphenyl)-1-piperidineacetic acid | 507/509 | $\delta_{(CDCl3)}$ 1.55 - 1.79 (5H, m), 1.86 - 2.00 (4H, m), 2.16 - 2.27 (4H, m), 2.39 - 2.74 (4H, m), 2.87 (1H, t), 3.37 (1H, d), 3.69 - 3.78 (1H, m), 3.87 (3H, s), 4.18 - 4.26 (1H, m), 5.03 (1H, s), 6.72 (1 H, dd), 6.91 - 7.03 (3H, m), 7.25 - 7.31 (1H, m), 7.37 (1H, t), 7.51 (1H, d) |
| 5 | 4-[[4-(3,4-Dichlorophenoxy)-1-piperidinyl]methyl]-α-(2-methylphenyl)-1-piperidineacetic acid | 491/493 | $\delta_{(CDCl3)}$ 1.47 - 1.75 (4H, m), 1.80 - 1.95 (5H, m), 2.12 - 2.23 (4H, m), 2.43 - 2.66 (6H, m), 2.76 - 2.90 (1H, m), 3.39 (1H, d), 3.49 (1H, s), 3.84 - 3.96 (1H, m), 4.14 - 4.25 (1H, m), 4.76 (1H, s), 6.72 (1H, dd), 6.96 (1H, d), 7.16 - 7.32 (4H, m), 7.78 (1H, d) |
| 6 | 4-[[4-(3,4-Dichlorophenoxy)-1-piperidinyl]methyl]-α-(4-methylphenyl)-1-piperidineacetic acid | 491/493 | $\delta_{(CDCl3)}$ 1.55 - 1.79 (5H, m), 1.81 - 1.96 (4H, m), 2.14 - 2.25 (4H, m), 2.35 (3H, s), 2.43 - 2.73 (4H, m), 2.76 - 2.87 (1H, m), 3.47 (1H, d), 3.68 - 3.77 (1H, m), 4.15 - 4.25 (1H, m), 4.54 (1H, s), 6.72 (1H, dd), 6.96 (1H, d), 7.18 (2H, d), 7.26 - 7.31 (1H, m), 7.42 (2H, d) |
| 7 | 4-[[4-(2,4-Dichloro-3-methylphenoxy)-1-piperidinyl]methyl]-α-(2-methoxyphenyl)-1-piperidineacetic acid | 521/523 | $\delta_{(CDCl3)}$ 1.55 - 1.68 (2H, m), 1.74 - 2.00 (5H, m), 2.16 - 2.28 (4H, m), 2.45 (3H, s), 2.57 - 2.90 (6H, m), 3.41 (1H, d), 3.66 - 3.77 (1H, m), 3.87 (3H, s), 4.24 - 4.35 (1H, m), 5.09 (1H, s), 6.72 (1H, d), 6.92 - 7.02 (2H, m), 7.17 (1H, d), 7.36 (1H, dd), 7.53 (1H, d) |
| 8 | 4-[[4-(2,4-Dichloro-3-methylphenoxy)-1-piperidinyl]methyl]-α-(2-methylphenyl)-l-piperidineacetic acid | 505/507 | $\delta_{(CDCl3)}$ 1.73 - 1.96 (8H, m), 2.14 - 2.28 (5H, m), 2.45 (3H, s), 2.53 (3H, s), 2.57 - 2.66 (4H, m), 2.75 - 2.86 (1H, m), 3.36 (1H, d), 3.80 - 3.91 (1H, m), 4.24 - 4.32 (1H, m), 4.73 (1H, s), 6.71 (1H, d), 7.14 - 7.24 (4H, m), 7.77 (1H, d) |

(continued)

| Example | Name | M+H | $^1$H NMR |
|---|---|---|---|
| 9 | 4-[[4-(2,4-Dichloro-3-methylphenoxy)-1-piperidinyl]methyl]-α-(4-methylphenyl)-1-piperidineacetic acid | 505/507 | $\delta_{(CDCl3)}$ 1.55 - 1.94 (9H, m), 2.14 - 2.27 (4H, m), 2.35 (3H, s), 2.45 (3H, s), 2.52 - 2.82 (5H, m), 3.46 (1H, d), 3.64 - 3.73 (1H, m), 4.24 - 4.32 (1H, m), 4.47 (1H, s), 6.71 (1H, d), 7.17 (3H, d), 7.43 (2H, d) |
| 10 | 4-[[4-(4-Chloro-2-methylphenoxy)-1-piperidinyl]methyl]-α-(2-methoxyphenyl)-1-piperidineacetic acid | 487/489 | $\delta_{(CDCl3)}$ 1.58 - 1.65 (2H, m), 1.70 - 1.80 (4H, m), 1.85 - 1.95 (4H, m), 2.15 - 2.26 (7H, m), 2.46 - 2.74 (3H, m), 2.80 - 2.91 (1H, m), 3.42 (1H, d), 3.68 - 3.77 (1H, m), 3.87 (3H, s), 4.19 - 4.28 (1H, m), 5.09 (1H, s), 6.70 (1H, d), 6.91 - 7.11 (4H, m), 7.36 (1H, dd), 7.53 (1H, d) |
| 11 | 4-[[4-(4-Chloro-2-methylphenoxy)-1-piperidinyl]methyl]-α-(2-methylphenyl)-1-piperidineacetic acid | 471/473 | $\delta_{(CDCl3)}$ 1.48 - 1.95 (11H, m), 2.13 (3H, s), 2.25 (2H, t), 2.46 - 2.90 (8H, m), 3.36 (1H, d), 3.83 - 3.93 (1H, m), 4.19 - 4.28 (1H, m), 4.78 (1H, s), 6.70 (1H, d), 7.02 - 7.11 (2H, m), 7.15 - 7.25 (3H, m), 7.74 (1H, d) |
| 12 | α-[4-[[4-(3,4-Dichlorophenoxy)-1-piperidinyl]methyl]-1-piperidinyl]-2,4-dimethoxy-5-pyrimidineacetic acid | 539/541 | $\delta(_{CDCl3})$ 1.48 - 2.80 (19H, m), 3.27 - 3.35 (1H, m), 3.51 (3H, s), 3.99 (3H, s), 4.29 - 4.37 (1H, m), 4.55 (1H, s), 6.73 (1H, dd), 6.98 (1H, d), 7.31 (1H, d), 8.06 (1H, s) |
| 13 | 4-[[4-(4-Chloro-2-methylphenoxy)-1-piperidinyl]methyl]-α-(4-methylphenyl)-1-piperidineacetic acid | 471/473 | $\delta_{(CDCl3)}$ 1.56-1.79 (5H,m), 1.83-1.94 (4H, m), 2.13-2.27 (7H, m), 2.35 (3H, s), 2.44 - 2.95 (5H, m), 3.48 (1H, d), 3.71 (1H, d), 4.19 - 4.28 (1H, m), 4.50 (1H, s), 6.70 (1H, d), 7.03 - 7.11 (2H, m), 7.18 (2H, d), 7.43 (2H, d) |
| 14 | α-[3-(Acetylamino)phenyl]-4-[[4-(3,4-dichlorophenoxy)-1-piperidinyl]methyl]-1-piperidineacetic acid | 534/536 | $\delta_{(CDCl3)}$ 1.46 - 1.64 (2H, m), 1.68 - 1.83 (3H, m), 1.87 - 1.97 (2H, m), 2.05 - 2.25 (7H, m), 2.36 - 2.77 (8H, m), 3.33 - 3.68 (2H, m), 4.22 (1H, s), 4.41 (1H, s), 6.73 (1H, dd), 6.96 - 7.02 (2H, m), 7.22 - 7.33 (2H, m), 7.49 (1H, s), 8.09 (1H, d) |
| 15 | 4-[[4-(2,4-Dichloro-3-methylphenoxy)-1-piperidinyl]methyl]-α-(4-fluorophenyl)-1-piperidineacetic acid | 509/511 | $\delta_{(CDCl3)}$ 1.54 - 2.47 (18H, m), 2.61 - 2.72 (2H, m), 3.28 - 3.35 (1H, m), 3.54 - 3.61 (1H, m), 4.29 - 4.42 (2H, m), 6.71 (1H, d), 7.05 (2H, t), 7.17 (1H, d), 7.54 (2H, dd) |
| 16 | 4-[[4-(2,4-Dichlorophenoxy)-1-piperidinyl]methyl]-α-phenyl-1-piperidineacetic acid | 477/479 | $\delta_{(CD3OD)}$ 1.48 - 1.61 (2H, m), 1.75 - 1.89 (4H, m), 1.90 - 2.04 (4H, m), 2.28 (2H, d), 2.33 - 2.41 (2H, m), 2.67 - 2.77 (3H, m), 2.96 (2H, t), 4.37 - 4.50 (2H, m), 7.07 (1H, d), 7.22 (1H, dd), 7.39 (1H, d), 7.42 - 7.46 (3H, m), 7.54 - 7.58 (2H, m) |

(continued)

| Example | Name | M+H | $^1$H NMR |
|---|---|---|---|
| 17 | 4-[[4-(3,4-Dichlorophenoxy)-1-piperidinyl]methyl]-4-hydroxy-α-phenyl-1-piperidineacetic acid | 493/495 | $\delta_{(CD3OD)}$ 1.64- 1.82 (4H, m), 1.85 - 2.03 (3H, m), 2.40 (2H, s), 2.49 - 2.57 (2H, m), 2.81 - 2.90 (3H, m), 2.98 - 3.16 (2H, m), 3.51 - 3.72 (2H, m), 4.32 - 4.39 (1H, m), 4.52 (1H, s), 6.87 (1H, dd), 7.08 (1H, d), 7.37 (1H, d), 7.42 - 7.47 (3H, m), 7.55-7.60 (2H, m) |
| 18 | 4-[[4-(3,4-Dichlorophenoxy)-1-piperidinyl]methyl]-4-hydroxy-α-(4-methylphenyl)-1-piperidineacetic acid | 507/509 | $\delta_{(CD30D)}$ 1.64 - 1.81 (4H, m), 1 .82 - 2.02 (4H, m), 2.36 (3H, s), 2.40 (2H, s), 2.49 - 2.57 (2H, m), 2.81 - 2.89 (2H, m), 2.92 - 3.06 (2H, m), 3.62 - 3.75 (2H, m), 4.32 - 4.39 (1H, m), 4.47 (1H, s), 6.87 (1H, dd), 7.08 (1H, d), 7.26 (2H, d), 7.36 (1H, d), 7.44 (2H, d) |
| 19 | 4-[[4-(3,4-Dichlorophenoxy)-1-piperidinyl]methyl]-4-hydroxy-α-(2-methoxyphenyl)-1-piperidineacetic acid | 523/525 | $\delta_{(CD3OD)}$ 1.65 - 1.80 (4H, m), 1.93 - 2.04 (4H, m), 2.40 (2H, s), 2.48 - 2.57 (2H, m), 2.81 - 2.89 (2H, m), 2.94 - 3.06 (2H, m), 3.67 - 3.77 (2H, m), 3.91 (3H, s), 4.32 - 4.39 (1H, m), 4.98 (1H, s), 6.87 (1H, dd), 7.03 (1H, td), 7.08 (1H, d), 7.09 - 7.12 (1 H, m), 7.36 (1H, d), 7.41 - 7.46 (1H, m), 7.53 (1H, dd) |
| 21 | 4-[[4-(2,6-Dichlorophenoxy)-1-piperidinyl]methyl]-α-phenyl-1-piperidineacetic acid | 477/479 | $\delta_{(CD30D)}$ 1.37 - 1.53 (2H, m), 1.67 - 1.80 (4H, m), 1.81 - 1.96 (3H, m), 2.19 (2H, d), 2.24 - 2.35 (2H, m), 2.56 - 2.74 (3H, m), 2.80 - 2.92 (2H, m), 3.61 - 3.80 (1H, m), 4.28 - 4.35 (1H, m), 4.38 - 4.46 (1H, m), 6.94 (1H, dd), 6.99 (1H, dd), 7.10 (1H, t), 7.31 - 7.37 (3H, m), 7.43 - 7.50 (2H, m) |
| 26 | (S)-4-[[4-(3,4-Dichlorophenoxy)-1-piperidinyl]methyl]-α-phenyl-piperidineacetic acid | 477/479 | $\delta_{(CD3OD + N\alpha O\Delta)}$ 1.25 - 1.38 (2H, m), 1.44 - 1.62 (2H, m), 1.68 - 2.06 (8H, m), 2.27 - 2.41 (4H, m), 2.69 - 2.78 (2H, m), 2.97 (1H, t), 4.35 - 4.47 (2H, m), 6.87 (1H, dd), 7.09 (1H, d), 7.37 (1H, d), 7.44 (3H, t), 7.54 - 7.59 (2H, m) |
| 28 | Methyl4-[[4-(2,4-dichloro-3-methylphenoxy)-1-piperidinyl] methyl]-a-phenyl-1-piperidineacetate ($\alpha^1$*R*)- | 505/507 | |
| 29 | ($\alpha^1$*S*)-1,1-Dimethylethyl 4-[[4-(2,4-dichloro-3-methylphenoxy)-1-piperidinyl]methyl]-α-phenyl-1-piperidineacetate | 547/549 | |
| 30 | ($\alpha^1$*R*)- Methyl 4-[[4-(3,4-dichloro-2-methylphenoxy)-1-piperidinyl]methyl]-α-phenyl-1-piperidineacetate | 505/507 | |
| 31 | ($\alpha^1$*R*)- Methyl 4-[[4-(3,4-dichloro-2-methylphenoxy)-1-piperidinyl]methyl]-α-phenyl-1-piperidineacetate | 505/507 | |

(continued)

| Example | Name | M+H | $^1$H NMR |
|---|---|---|---|
| 32 | Methyl (S)- 4-[[4-(3,4-dichlorophenoxy)-1-piperidinyl]methyl]-α-(1-methylethyl)-1-piperidineacetate | 457/459 | |
| 33 | 1,1-Dimethylethyl 4-[[4-(3,4-dichlorophenoxy)-1-piperidinyl]methyl]-α,α-dimethyl-1-piperidineacetate | 485/487 | |
| 35 | (α$^1$*R*)-4-[[4-(2,4-Dichloro-3-methylphenoxy)-1-piperidinyl]methyl]-α-phenyl-1-piperidineacetic acid | 491/493 | $\delta_{(CD3OD)}$ 1.43 - 1.63 (2H, m), 1.78 - 1.89 (4H, m), 1.91 - 2.05 (3H, m), 2.15 (1H, s), 2.32 (2H, d), 2.38 - 2.48 (5H, m), 2.69 - 2.80 (3H, m), 2.91 - 3.05 (2H, m), 4.40 - 4.52 (2H, m), 6.94 (1H, d), 7.25 (1H, d), 7.43 - 7.45 (3H, m), 7.55 - 7.58 (2H, m) |
| 36 | (α$^1$*S*)- 4-[[4-(2,4-Dichloro-3-methylphenoxy)-1-piperidinyl] methyl]-(α-phenyl-1-piperidineacetic acid | 491/493 | $\delta_{(CD3OD)}$ 1.42 - 1.62 (2H, m), 1.77 - 1.90 (4H, m), 1.90 - 2.05 (4H, m), 2.30 (2H, d), 2.35 - 2.45 (5H, m), 2.72 (3H, t), 2.97 (2H, t), 4.40 - 4.49 (2H, m), 6.93 (1H, d), 7.25 (1H, d), 7.42 - 7.46 (3H, m), 7.53 - 7.59 (2H, m) |
| 37 | (α$^1$*R*)-4-[[4-(3,4-Dichloro-2-methylphenoxy)-1 piperidinyl]methyl]-α-phenyl-1-piperidineacetic acid | 491/493 | $\delta_{(CD3OD)}$ 1.43 - 1.67 (2H, m), 1.76 - 1.94 (4H, m), 1.95 - 2.10 (4H, m), 2.30 - 2.37 (5H, m), 2.45 (2H, t), 2.75 (3H, t), 3.01 (2H, t), 4.41 - 4.52 (2H, m), 6.93 (1H, d), 7.30 (1H, d), 7.47 (3H, dd), 7.59 (2H, q) |
| 38 | (α$^1$*S*)-4-[[4-(3,4-Dichloro-2-methylphenoxy)-1-piperidinyl]methyl]-α-phenyl-1-piperidineacetic acid | 491/493 | $\delta_{(CD3OD)}$ 1.30 - 1.54 (2H, m), 1.65 - 1.81 (4H, m), 1.81 - 1.98 (4H, m), 2.19 - 2.25 (5H, m), 2.28 - 2.39 (2H, m), 2.57 - 2.70 (3H, m), 2.82 - 2.98 (2H, m), 4.29 - 4.40 (2H, m), 6.81 (1H, d), 7.18 (1H, d), 7.32 - 7.39 (3H, m), 7.43 - 7.51 (2H, m) |
| 39 | (*S*)-4-[[4-(3,4-Dichlorophenoxy)-1-piperidinyl]methyl]-α-(1-methylethyl)-1-piperidineacetic acid | 443/445 | $\delta_{(CD3OD + N\alpha O\Delta)}$ 1.02 (3H, d), 1.14 (3H, d), 1.26 - 1.38 (3H, m), 1.42 - 1.64 (2H, m), 1.73 - 1.85 (2H, m), 1.98 - 2.07 (4H, m), 2.28 - 2.39 (2H, m), 2.40 - 2.49 (2H, m), 2.75 - 2.84 (2H, m), 2.97 - 3.04 (2H, m), 3.46 - 3.58 (2H, m), 4.39 - 4.46 (1H, m), 6.89 (1H, dd), 7.10 (1H, d), 7.38 (1H, d) |
| 40 | 4-[[4-(3,4-Dichlorophenoxy)-1-piperidinyl]methyl]-α,α-dimethyl-1-piperidineacetic acid | 429/431 | $\delta_{(CD3OD+NaOD)}$ 1.17 - 1.55 (8H, m), 1.61 - 1.77 (2H, m), 1.77 - 2.01 (7H, m), 2.23 - 2.40 (3H, m), 2.63 - 2.75 (2H, m), 2.88 (2H, t), 3.31 - 3.42 (1H; m), 4.28 - 4.39 (1H, m), 6.79 (1H, dd), 7.01 (1H, d), 7.28 (1H, d) |
| 43 | methyl 2-[(4-{[4-(2,4-Dichlorophenoxy) piperidin-1-yl]methyl}piperidin-1-yl) methyl]benzoate | 491/493 | |

(continued)

| Example | Name | M+H | $^{1}$H NMR |
|---|---|---|---|
| 51 | 2-[(4-{[4-(3,4-dichlorophenoxy) piperidin-1-yl]methyl}piperidin-1-yl) methyl]benzoic acid | 477/479 | $\delta_{(CD3OD)}$ 1.30 - 1.46 (2H, m), 1.70 - 1.83 (3H, m), 1.95 - 2.11 (4H, m), 2.25 - 2.41 (4H, m), 2.69 - 2.79 (2H, m), 2.94 (2H, t), 3.31 - 3.41 (2H, m), 4.26 (2H, s), 4.41 (1 H, dt), 6.90 (1 H, dd), 7.11 (1 H, d), 7.39 (2H, d), 7.51 (2H, dtd), 7.97 (1H, dd) |
| 52 | 2-[[4-[[4-(2,4-Dichlorophenoxy)-1-piperidinyl]methyl]-1-piperidinyl] methyl]-benzoic acid | 477/479 | $\delta_{(CD3OD+NaOD)}$ 1.19 - 1.34 (2H, m), 1.47 - 1.61 (1H, m), 1.69 - 1.76 (2H, m), 1.76 - 1.86 (2H, m), 1.93 - 2.08 (4H, m), 2.22 (2H, d), 2.29 - 2.37 (2H, m), 2.64 - 2.72 (2H, m), 2.89 - 2.95 (2H, m), 3.83 (2H, s), 4.41 - 4.48 (1H, m), 7.07 (1H, d), 7.16 - 7.28 (3H, m), 7.38 (1H, d), 7.41 - 7.45 (2H, m) |
| 57 | 2-[[4-[[4-(3,4-Dichlorophenoxy)-1-piperidinyl]methyl]-1-piperidinyl] methyl]-5-fluoro-benzoic acid | 495/497 | $\delta_{(CD3OD)}$ 1.24 - 1.40 (2H, m), 1.66 - 1.77 (2H, m), 1.81 - 1.99 (5H, m), 2.34 (2H, d), 2.43 (2H, t), 2.71 - 2.79 (2H, m), 2.86 (2H, t), 3.22 - 3.29 (2H, m), 4.15 (2H, s), 4.31 - 4.39 (1H, m), 6.80 (1H, dd), 7.01 (1H, d), 7.11 (1H, td), 7.29 (1H, d), 7.31 (1H, dd), 7.56 (1H, dd) |
| 58 | 4-[[4-[[4-(2,4-Dichlorophenoxy)-1-piperidinyl]methyl]-1-piperidinyl] methyl]-3-fluoro-benzoic acid | 495/497 | $\delta_{(CD30D)}$ 1.30 - 1.49 (2H, m), 1.82 - 1.96 (3H, m), 2.00 - 2.07 (2H, m), 2.11 - 2.22 (2H, m), 2.47 - 2.58 (2H, m), 2.69 (2H, d), 2.86 - 2.97 (2H, m), 3.03 - 3.22 (4H, m), 3.95 (2H, s), 4.60 - 4.69 (1H, m), 7.14 (1H, d), 7.28 (1H, dd), 7.44 - 7.51 (2H, m), 7.68 (1H, dd), 7.78 (1H, dd) |
| 59 | 2-[[4-[[4-(3,4-Dichlorophenoxy)-1-piperidinyl]methyl]-1-piperidinyl] methyl]-4-oxazolecarboxylic acid, | 468/470 | $\delta_{(CD3OD)}$ 1.20 - 1.32 (2H, m), 1.77 (3H, d), 1.92 - 2.00 (2H, m), 2.08 - 2.15 (4H, m), 2.81 (2H, d), 2.90 (2H, d), 3.11 - 3.22 (4H, m), 3.64 (2H, s), 4.55 (1H, s), 6.85 (1H, dd), 7.10 (1 H, d), 7.31 (1H, d), 8.06 (1 H, s) |
| 60 | 4-[[4-[[4-(3,4-Dichlorophenoxy)-1-piperidinyl]methyl]-1-piperidinyl] methyl]-benzoic acid | 477/479 | $\delta_{(CD3OD)}$ 1.29 (2H, q), 1.70 - 1.83 (5H, m), 1.93 - 2.01 (2H, m), 2.40 (2H, d), 2.44 - 2.58 (4H, m), 2.82 (2H, tt), 3.12 (2H, d), 3.88 (2H, s), 4.34 - 4.43 (1H, m), 6.81 (1H, dd), 7.03 (1H, d), 7.29 (1H, d), 7.33 (2H, d), 7.87 (2H, d) |
| 61 | 3-[[4-[[4-(3,4-Dichlorophenoxy)-1-piperidinyl]methyl]-1-piperidinyl] methyl]-benzoic acid | 477/479 | $\delta_{(CD30D)}$ 1.25 - 1.41 (2H, m), 1.66 - 1.78 (3H, m), 1.82 - 1.99 (4H, m), 2.32 (2H, d), 2.37 - 2.47 (2H, m), 2.59 - 2.79 (4H, m), 3.25 (2H, s), 4.00 (2H, s), 4.30 - 4.39 (1H, m), 6.79 (1H, dd), 7.01 (1H, d), 7.28 (1H, d), 7.31 - 7.42 (2H, m), 7.87 - 7.92 (2H, m) |

(continued)

| Example | Name | M+H | $^1$H NMR |
|---|---|---|---|
| 62 | 2-[[4-[[4-(2,4-Dichloro-3-methylphenoxy)-1-piperidinyl]methyl]-1-piperidinyl]methyl]-benzoic acid | 491/493 | $\delta_{(CD30D)}$ 1.31 - 1.50 (2H, m), 1.81 - 1.93 (3H, m), 1.96 - 2.11 (4H, m), 2.39 (2H, d), 2.45 - 2.55 (5H, m), 2.77 - 2.85 (2H, m), 2.90 - 3.03 (2H, m), 3.34 - 3.40 (2H, m), 4.27 (2H, s), 4.46 - 4.54 (1H, m), 6.97 (1H, d), 7.28 (1H, d), 7.38 - 7.41 (1H, m), 7.51 (2H, dtd), 7.98 (1H, dd) |
| 63 | 2-[[4-[[4-(3,4-Dichloro-2-methylphenoxy)-1-piperidinyl]methyl]-1-piperidinyl]methyl]-benzoic acid | 491/493 | $\delta_{(CD3OD)}$ 1.26 - 1.42 (2H, m), 1.74 - 1.85 (3H, m), 1.92 - 2.09 (4H, m), 2.24 - 2.32 (2H, m), 2.30 (3H, s), 2.38 (2H, t), 2.69 (2H, t), 2.91 (2H, t), 3.30 - 3.39 (2H, m), 4.19 - 4.26 (2H, m), 4.37 - 4.47 (1H, m), 6.90 (1H, d), 7.26 (1H, d), 7.36 (1H, d), 7.48 (2H, quintetd), 7.95 (1H, d) |
| 64 | [2-[[4-[[4-(2,4-Dichloro-3-methylphenoxy)-1-piperidinyl]methyl]-1-piperidinyl]methyl]phenoxy]-acetic acid | 521/523 | $\delta(_{CD3OD})$ 1.46 - 1.63 (2H, m), 1.69 - 1.95 (7H, m), 2.23 - 2.29 (2H, m), 2.31 - 2.40 (5H, m), 2.63 - 2.73 (2H, m), 2.79 - 2.91 (2H, m), 3.35 (2H, d), 4.11 (2H, s), 4.34 - 4.42 (1H, m), 4.57 (2H, s), 6.85 (1H, d), 6.94 (1H, t), 7.07 (1H, d), 7.16 (1H, d), 7.24 (1H, d), 7.35 (1H, t) |
| 65 | 2-[[4-[[4-(2,4-Dichlorophenoxy)-1-piperidinyl]methyl]-1-piperidinyl] methyl]-benzenesulfonic acid | (ES -ve) 511/513 | $\delta_{(CD3OD)}$ 1.18 - 1.40 (2H, m), 1.69 - 1.82 (3H, m), 1.89 - 2.02 (4H, m), 2.25 (2H, d), 2.31 - 2.41 (2H, m), 2.63 - 2.73 (2H, m), 2.98 (2H, t), 3.34 (2H, d), 4.34 - 4.44 (1H, m), 4.52 (2H, s), 6.98 (1H, d), 7.14 (1H, dd), 7.30 (1H, d), 7.39 - 7.51 (3H, m), 7.91 - 7.96 (1H, m) |
| 66 | 2-[[4-[[4-(2,4-Dichloro-3-methylphenoxy)-1-piperidinyl]methyl]-1-piperidinyl]methyl]-benzenesulfonic acid | 527/529 | $\delta_{(CD3OD)}$ 1.15 - 1.33 (2H, m), 1.66 - 2.02 (7H, m), 2.12 - 2.37 (7H, m), 2.57 - 2.68 (2H, m), 2.88 - 3.02 (2H, m), 3.28 - 3.40 (2H, m), 4.31 - 4.40 (1H, m), 4.51 (2H, s), 6.84 (1H, d), 7.15 (1H, d), 7.39 - 7.50 (3H, m), 7.90 - 7.96 (1H, m) |
| 68 | Methyl 4-[[4-(2,4-dichloro-3-methylphenoxy)-1 piperidinyl]methyl]-1-piperidineacetate | 429/431 | |
| 69 | Methyl-4-[[4-(4-chloro-2-methylphenoxy)-1 piperidinyl]methyl]-1-piperidineacetate | (ES-ve) 393/395 | |
| 70 | Methyl4-[[4-(2,4-dichlorophenoxy)-1-piperidinyl]methyl]-1-piperidineacetate | 415/417 | |
| 71 | Methyl4-[[4-(3,4-dichloro-2-methylphenoxy)-1-piperidinyl]methyl]-1-piperidineacetate | 429/433 | |
| 72 | Methyl 4-[[4-(3,4-dichlorophenoxy)-1-piperidinyl]methyl]-4-hydroxy-1-piperidineacetate | 431/433 | |

(continued)

| Example | Name | M+H | $^1$H NMR |
|---|---|---|---|
| 74 | Methyl (2*S*)-2-(4-{[4-(3,4-dichlorophenoxy)piperidin-1-yl]methyl } piperidin-1-yl)propanoate | 429/431 | |
| 75 | (α*R*)-Methyl-4-[[4-(4-chloro-2--methylphenoxy)-1-piperidinyl]methyl]-α-methyl- 1 -piperidineacetate | (E*S*-ve) 407/409 | |
| 76 | (α*S*)-Methyl-4-[[4-(4-chloro-2--methylphenoxy)-1-piperidinyl]methyl]-α-methyl-1-piperidineacetate | (E*S*-ve) 407/409 | |
| 78 | 4-[[4-(2,4-Dichloro-3-methylphenoxy)-1-piperidinyl]methyl]-1-piperidineacetic acid | 415/417 | $\delta_{(CDCl3)}$ 1.45 - 1.61 (2H, m), 1.80- 1.93 (3H, m), 1.96 - 2.10 (4H, m), 2.33 (2H, d), 2.38 - 2.48 (2H, m), 2.47 (3H, s), 2.71 - 2.82 (2H, m), 3.01 (2H, t), 3.55 - 3.68 (2H, m), 3.59 (2H, s), 4.44 - 4.54 (1H, m), 6.96 (1H, d), 7.27 (1H, d) |
| 79 | 4-[[4-(4-Chloro-2-methylphenoxy)-1-piperidinyl]methyl]-1-piperidineacetic acid | | $\delta_{(CD3OD)}$ 1.45 - 1.61 (2H, m), 1.76 - 1.94 (3H, m), 1.97 - 2.09 (4H, m), 2.20 (3H, s), 2.32 (2H, d), 2.40 (2H, t), 2.69 - 2.78 (2H, m), 3.01 (2H, t), 3.57 - 3.66 (4H, m), 4.37 - 4.46 (1H, m), 6.89 (1H, d), 7.08-7.14 (2H, m) |
| 80 | [[4-(2,4-Dichlorophenoxy)-1-piperidinyl] methyl]-1-piperidineacetic acid | | $\delta_{(CD3OD)}$ 1.32 - 1.48 (2H, m), 1.67 - 1.80 (3H, m), 1.84 - 1.96 (4H, m), 2.20 (2H, d), 2.25 - 2.34 (2H, m), 2.59 - 2.68 (2H, m), 2.83 - 2.95 (2H, m), 3.45 - 3.55 (4H, m), 4.34 - 4.42 (1H, m), 6.98 (1H, d), 7.14 (1H, dd), 7.30 (1H, d) |
| 81 | 4-[[4-(3,4-Dichloro-2-methylphenoxy)-1-piperidinyl]methyl]-1-piperidineacetic acid | 415/417 | $\delta_{(CD3OD)}$ 1.52 (2H, dd), 1.79 - 1.94 (3H, m), 1.99 - 2.08 (4H, m), 2.32 (3H, s), 2.38 (2H, d), 2.48 (2H, t), 2.77 (2H, t), 3.01 (2H, t), 3.55 - 3.64 (4H, m), 4.41 - 4.50 (1H, m), 6.93 (1H, d), 7.28 (1H, d) |
| 82 | 4-[[4-(3,4-Dichlorophenoxy)-1-piperidinyl]methyl]-4-hydroxy-1-piperidineacetic acid | 417/419 | |
| 83 | (2*R*)-2-(4-{[4-(3,4-Dichlorophenoxy) piperidin-1-yl]methyl}piperidin-1-yl) propanoic acid | 415/417 | $\delta_{(CD3OD)}$ 1.42 - 1.59 (5H, m), 1.71 - 2.12 (7H, m), 2.28 - 2.41 (4H, m), 2.70 - 2.80 (2H, m), 2.93 - 3.14 (2H, m), 3.49 - 3.62 (3H, m), 4.37 - 4.46 (1H, m), 6.91 (1H, dd), 7.12 (1H, d), 7.40 (1H, t) |
| 84 | (2*S*)-2-(4-{[4-(3,4-Dichlorophenoxy) piperidin-1-yl]methyl}piperidin-1-yl) propanoic acid | 415/417 | $\delta_{(CD3OD)}$ 1.44 - 1.60 (5H, m), 1.73 - 2.12 (7H, m), 2.30 - 2.43 (4H, m), 2.71 - 2.81 (2H, m), 2.93 - 3.14 (2H, m), 3.50 - 3.62 (3H, m), 4.38 - 4.48 (1H, m), 6.91 (1H, dd), 7.12 (1H, d), 7.40 (1H, d) |
| 85 | (α*R*)-4-[[4-(4-Chloro-2-methylphenoxy)-1-piperidinyl]methyl]-α-methyl-1-piperidineacetic acid | | $\delta_{(CD3OD)}$ 1.45- 1.61 (5H, m), 1.76 - 2.13 (7H, m), 2.20 (3H, s), 2.32 (2H, d), 2.41 (2H, t), 2.69 - 2.79 (2H, m), 2.94 - 3.14 (2H, m), 3.50 - 3.62 (3H, m), 4.37 - 4.46 (1H, m), 6.90 (1H, d), 7.08 - 7.14 (2H, m) |

(continued)

| Example | Name | M+H | $^1$H NMR |
|---|---|---|---|
| 86 | (α*S*)-4-[[4-(4-Chloro-2-methylphenoxy)-1-piperidinyl]methyl]-α-methyl-1-piperidineacetic acid | | $\delta_{(CD3OD)}$ 1.45 - 1.55 (5H, m), 1.75 - 1.91 (3H, m), 1.95 - 2.09 (4H, m), 2.18 (3H, s), 2.28 (2H, d), 2.37 (2H, t), 2.67 - 2.74 (2H, m), 2.92 - 3.09 (2H, m), 3.49 - 3.58 (3H, m), 4.35 - 4.42 (1H, m), 6.87 (1H, d), 7.06 - 7.12 (2H, m) |
| 88 | Methyl-4-[[4-(4-chloro-2-methylphenoxy)-1-piperidinyl]methyl]-α,α-dimethyl-1- piperidinepropanoate | 437/439 | |
| 89 | Methyl4-[[4-(2,4-dichloro-3-methylphenoxy)-1-piperidinyl]methyl]-α,α-dimethyl-1-piperidinepropanoate | 471/473 | |
| 91 | 4-[[4-(4-Chloro-2-methylphenoxy)-1-piperidinyl]methyl]-α,α-dimethyl-1-piperidinepropanoic acid | 423/425 | $\delta_{(CD3OD)}$ 1.14 (6H, s), 1.32 - 1.47 (2H, m), 1.65 - 1.80 (3H, m), 1.92 (4H, d), 2.08 (3H, s), 2.22 (2H, d), 2.27 - 2.36 (2H, m), 2.59 - 2.68 (2H, m), 2.86 - 2.99 (4H, m), 3.37 - 3.45 (2H, m), 4.26 - 4.34 (1H, m), 6.78 (1H, d), 6.97 (1H, d), 7.01 (1H, q) |
| 92 | 4-[[4-(2,4-Dichloro-3-methylphenoxy)-1-piperidinyl]methyl]-α,α-dimethyl-1-piperidinepropanoic acid | 457/459 | $\delta_{(CD3OD + N\alpha O\Delta)}$ 1.10 (6H, s), 1.21 - 1.36 (4H, m), 1.59 - 1.70 (2H, m), 1.77 - 1.87 (2H, m), 1.93 - 2.02 (2H, m), 2.04 - 2.13 (2H, m), 2.18 - 2.23 (2H, m), 2.29 - 2.38 (1H, m), 2.44 (3H, s), 2.48 (2H, s), 2.64 - 2.73 (2H, m), 2.87 - 2.93 (2H, m), 4.41 4.48 (1H, m), 6.94 (1H, d), 7.24 (1H, d) |
| 95 | Methyl 4-[[4-(3,4-dichlorophenoxy)-1-piperidinyl]methyl]- 1-piperidinepentanoate | 457/459 | |
| 96 | Ethyl4-[[4-(3,4-dichlorophenoxy)-1-piperidinyl]methyl]-1-piperidinehexanoate | 485/487 | |
| 97 | 4-[[4-(3,4-Dichlorophenoxy)-1-piperidinyl]methyl]-1-piperidinebutanoic acid | 429/431 | $\delta_{(CD3OD+NaOD)}$ 1.08 - 1.26 (2H, m), 1.42 - 1.53 (1H, m), 1.61 - 1.77 (6H, m), 1.85 - 1.97 (4H, m), 2.05 (2H, t), 2.13 (2H, d), 2.17 - 2.24 (2H, m), 2.24 - 2.32 (2H, m), 2.56 - 2.66 (2H, m), 2.87 (2H, d), 4.24 - 4.32 (1H, m), 6.78 (1H, dd), 6.99 (1H,d), 7.27 (1H,d) |
| 98 | 4-[[4-(3,4-Dichlorophenoxy)-1-piperidinyl]methyl]-1-piperidinepentanoic acid | 443/445 | $\delta_{(CD3OD+NaOD)}$ 1.15-1.29 (1H, m), 1.36 (2H, q), 1.55 - 1.62 (2H, m), 1.62 - 1.72 (4H, m), 1.86 - 1.98 (4H, m), 2.16 (2H, t), 2.22 (2H, d), 2.28 (2H, t), 2.61 - 2.70 (2H, m), 2.78 (2H, t), 2.93 (2H, t), 3.36 - 3.44 (2H, m), 4.27 - 4.34 (1H, m), 6.79 (1H, dd), 7.00 (1H, d), 7.28 (1H, d) |

(continued)

| Example | Name | M+H | $^1$H NMR |
|---|---|---|---|
| 99 | 4-[[4-(3,4-Dichlorophenoxy)-1-piperidinyl]methyl]-1-piperidinehexanoic acid | 457/459 | $\delta_{(CD3OD+NaOD)}$ 1.22 - 1.42 (4H, m), 1.49-1.71 (5H, m), 1.74 - 1.85 (4H, m), 1.94 - 2.07 (4H, m), 2.14 - 2.27 (4H, m), 2.29 - 2.40 (4H, m), 2.67 - 2.77 (2H, m), 2.93 - 3.01 (2H, m), 4.35-4.44 (1H, m), 6.90 (1H, dd), 7.10 (1H, d), 7.39 (1H,d) |
| 101 | 4-(3,4-Dichlorophenoxy)-1-[[1-[2-(2*H*-tetrazol-5-yl)ethyl]-4-piperidinyl]methyl]-piperidine | 439/441 | $\delta_{(CD3OD)}$ 1.35 (2H, dd), 1.66 - 1.76 (2H, m), 1.77 - 1.84 (1H, m), 1.88 - 1.99 (4H, m), 2.31 (2H, d), 2.34 - 2.46 (2H, m), 2.66 - 2.89 (4H, m), 3.12- 3.18 (2H, m), 3.24 - 3.36 (2H, m), 3.45 (2H, d), 4.19 - 4.43 (1H, m), 6.80 (1H, dd), 7.02 (1H, d), 7.29 (1H, d) |
| 103 | *N*-[3-[4-[[4-(3,4-Dichlorophenoxy)-1-piperidmyl]methyl]-1-piperidinyl]propyl]-1,1,1-trifluoro-methanesulfonamide | 532/534 | $\delta_{(CD3OD)}$ 1.14 - 1.30 (2H, m), 1.53 - 1.76 (5H, m), 1.80 (2H, d), 1.86 - 1.97 (2H, m), 2.18 (2H, d), 2.21 - 2.34 (4H, m), 2.64 (4H, t), 3.10 (2H, d), 3.17 (2H, t), 4.20 - 4.40 (1H, m), 6.79 (1H, dd), 7.00 (1H, d), 7.28 (1H, d) |
| 104 | *N*-[2-[4-[[4-(3,4-Dichloro-2-methylphenoxy)-1-piperidinyl]methyl]-1-piperidinyl]ethyl]-1,1,1-trifluoro-methanesulfonamide, | 532/534 | $\delta_{(CD3OD)}$ 1.27 - 1.43 (2H, m), 1.69 - 1.81 (1H, m), 1.82 - 1.92 (4H, m), 1.99 - 2.10 (2H, m), 2.32 (3H, s), 2.39 - 2.48 (4H, m), 2.51 - 2.62 (2H, m), 2.77 (2H, t), 2.80 - 2.89 (2H, m), 3.19 (2H, d), 3.36 (2H, t), 4.43 - 4.53 (1H, m), 6.92 (1H, d), 7.28 (1H, d) |

EXAMPLE 105

Pharmacological Analysis: Calcium flux $[Ca^{2+}]_i$ assay

Human eosinophils

Human eosinophils were isolated from EDTA anticoagulated peripheral blood as previously described (Hansel et al., J. Immunol. Methods, 1991, 145, 105-110). The cells were resuspended ($5x10^6$ $mL^{-1}$) and loaded with 5μM FLUO-3/AM + Pluronic F127 2.2μl/ mL (Molecular Probes) in low potassium solution (LKS; NaCl 118mM, $MgSO_4$ 0.8mM, glucose 5.5mM, $Na_2CO_3$ 8.5mM, KCl 5mM, HEPES 20mM, $CaCl_2$ 1.8mM, BSA 0.1 %, pH 7.4) for one hour at room temperature. After loading, cells were centrifuged at 200g for 5min and resuspended in LKS at $2.5x10^6$ $mL^{-1}$. The cells were then transferred to 96 well FLIPr plates (Poly-D-Lysine plates from Becton Dickinson pre-incubated with 5μM fibronectin for two hours) at 25μl/well. The plate was centrifuged at 200g for 5min and the cells were washed twice with LKS (200μl; room temperature).

A compound of the Examples was pre-dissolved in DMSO and added to a final concentration of 0.1%(v/v) DMSO. Assays were initiated by the addition of an $A_{50}$ concentration of eotaxin and the transient increase in fluo-3 fluorescence ($1_{EX}$ =490nm and $1_{Em}$ = 520nm) monitored using a FLIPR (Fluorometric Imaging Plate Reader, Molecular Devices, Sunnyvale, U.S.A.).

Compounds of the Examples were found to be antagonists if the increase in fluorescence induced by eotaxin (a selective CCR3 agonist) was inhibited in a concentration dependent manner. The concentration of antagonist required to inhibit the fluorescence by 50% can be used to determine the $IC_{50}$ for the antagonist at the CCR3 receptor.

EXAMPLE 106

Human eosinophil chemotaxis

Human eosinophils were isolated from EDTA anticoagulated peripheral blood as previously described (Hansel et al., J. Immunol. Methods, 1991, 145, 105-110). The cells were resuspended at $10x10^6$ $mL^{-1}$ in RPMI containing 200 IU/ mL penicillin, 200 μg/ mL streptomycin sulfate and supplemented with 10% HIFCS, at room temperature.

Eosinophils (700 μl) were pre-incubated for 15 mins at 37° C with 7 μl of either vehicle or compound (100x required final concentration in 10% DMSO). The chemotaxis plate (ChemoTx, 3μm pore, Neuroprobe) was loaded by adding 28μl of a concentration of eotaxin 0.1 to 100nM (a selective CCR3 agonist over this concentration range) containing a concentration of a compound according to the Examples or solvent to the lower wells of the chemotaxis plate. The filter was then placed over the wells and 25 μl of eosinophil suspension were added to the top of the filter. The plate was incubated for 1 hr at 37° C in a humidified incubator with a 95% air/5% $CO_2$ atmosphere to allow chemotaxis.

The medium, containing cells that had not migrated, was carefully aspirated from above the filter and discarded. The filter was washed once with phosphate buffered saline (PBS) containing 5 mM EDTA to remove any adherent cells. Cells that had migrated through the filter were pelleted by centrifugation (300xg for 5 mins at room temperature) and the filter removed and the supernatant transferred to each well of a 96-well plate (Costar). The pelleted cells were lysed by the addition of 28 μl of PBS containing 0.5% Triton x 100 followed by two cycles of freeze/thawing. The cell lysate was then added to the supernatant. The number of eosinophils migrating was quantified according to the method of Strath et al., J. Immunol. Methods, 1985, 83, 209 by measuring eosinophil peroxidase activity in the supernatant.

Compounds of the Examples were found to be antagonists of eotaxin mediated human eosinophil chemotaxis if the concentration response to eotaxin was shifted to the right of the control curve. Measuring the concentration of eotaxin required to give 50% chemotaxis in the presence or absence of compounds enables the apparent affinity of the compounds at CCR3 to be calculated.

| Example | % inhibition at 1μm |
|---|---|
| 1 | 96 |
| 4 | 90 |
| 10 | 108 |
| 13 | 87 |

EXAMPLE 107

Guinea-pig isolated trachea

(See for example, Harrison, R.W.S., Carswell, H. & Young, J.M. (1984) European J. Pharmacol., 106,405-409.)

Male albino Dunkin-Hartley guinea-pigs (250g) were killed by cervical dislocation and the whole trachea removed. After clearing the adherent connective tissue, the trachea was cut into six ring segments each three cartilage bands wide and then suspended in 20 mL organ baths containing Krebs-Henseleit solution of the following composition (mM): NaCl 117.6, $NaH_2PO_4$ 0.9, $NaHCO_3$ 25.0, $MgSO_4$ 1.2, KCl 5.4, $CaCl_2$ 2.6 and glucose 11.1. The buffer was maintained at 37°C and gassed with 5% $CO_2$ in oxygen. Indomethacin (2.8μM) was added to the Krebs solution to prevent development of smooth muscle tone due to the synthesis of cyclooxygenase products. The tracheal rings were suspended between two parallel tungsten wire hooks, one attached to an Ormed beam isometric force transducer and the other to a stationary support in the organ bath. Changes in isometric force were recorded on 2-channel Sekonic flat bed chart recorders.

Experimental protocols

At the beginning of each experiment a force of 1g was applied to the tissues and this was reinstated over a 60 minute equilibration period until a steady resting tone was achieved. Subsequently, a cumulative histamine concentration effect (E/[A]) curve was constructed at 0.5 $log_{10}$ unit increments, in each tissue. The tissues were then washed and approximately 30 minutes later, test compound or vehicle (20% DMSO) was added. Following an incubation period of 60 minutes a second E/[A] curve was performed to histamine.

Contraction responses were recorded as a percentage of the first curve maximum.

Data analysis

Experimental E/[A] curve data were analysed for the purposes of estimating the potencies (p[$A_{50}$] values) of histamine in the absence and presence of the test compound. Affinity ($pA_2$) values of test compounds were subsequently calculated using the following equation:

$$\log(r-1) = \log[B] + pA_2$$

where r = $[A]_{50}$ in presence of test compound/$[A]_{50}$ in absence of antagonist and [B] is the concentration of test compound. Compounds of the Examples were found to be H1 antagonists.

EXAMPLE 108

Histamine H1 receptor binding activity of compounds of the invention was assessed by competition displacement of 1nM [3H]-pyrilamine (Amersham, Bucks, Product code TRK 608, specific activity 30Ci/mmol) to 2μg membranes prepared from recombinant CHO-K1 cells expressing the human H1 receptor (Euroscreen SA, Brussels, Belgium, product code ES-390-M) in assay buffer (50mM Tris pH 7.4 containing 2mM $MgCl_2$, 250mM sucrose and 100mM NaCl) for 1 hour at room temperature.

| Example | H1 pKi /[1328_S] |
|---|---|
| 1 | 7.2 |
| 2 | 7.5 |
| 3 | 7.4 |
| 4 | 7.0 |
| 5 | 7.1 |
| 6 | 7.7 |
| 7 | 7.1 |
| 8 | 7.3 |
| 9 | 7.5 |
| 10 | 6.6 |
| 11 | 6.8 |
| 12 | 6.7 |
| 13 | 7.6 |
| 14 | 7.6 |
| 15 | 7.6 |
| 17 | 8.0 |
| 18 | 7.8 |
| 19 | 8.1 |
| 42 | 8.0 |
| 69 | 6.9 |
| 77 | 6.9 |
| 78 | 6.7 |
| 82 | 7.0 |
| 84 | 6.7 |

(continued)

| Example | H1 pKi /[1328_S] |
|---|---|
| 100 | 7.4 |
| 103 | 7.7 |

Scheme 1

To prepare compounds of formula (I) wherein Z is $CO_2R^3$

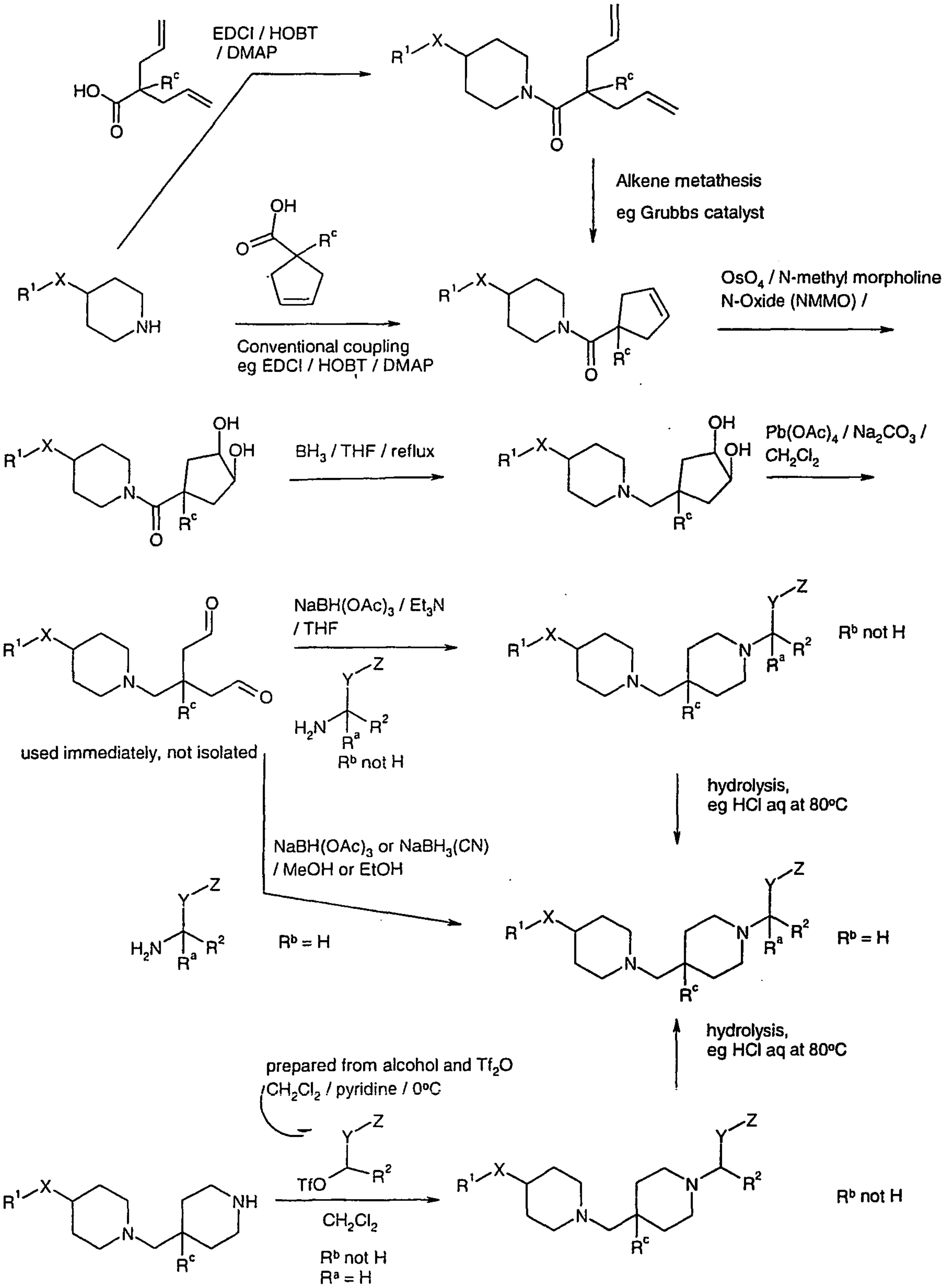

## Claims

**1.** A compound of formula (I):

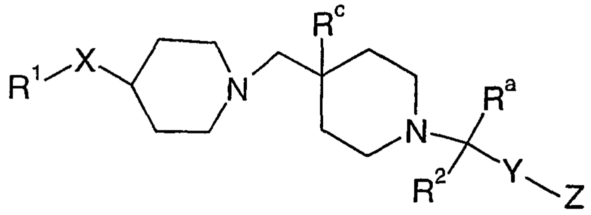

(I)

wherein:

X is $CH_2$, C(O), O, S, S(O), $S(O)_2$ or $NR^3$;
Y is a bond, $C_{1-6}$ alkylene (optionally substituted by $C_{1-4}$ alkyl or phenyl), phenylene (optionally substituted by halogen, hydroxy, $C_{1-4}$ alkyl or $C_{1-4}$ alkoxy) or heterocyclylene (optionally substituted by halogen, hydroxy, $C_{1-4}$ alkyl or $C_{1-4}$ alkoxy);
Z is $CO_2R^b$, $NHS(O)_2CF_3$, $S(O)_2OH$, $OCH_2CO_2R^b$ or tetrazolyl;
$R^1$ is hydrogen, $C_{1-6}$ alkyl, aryl or heterocyclyl;
$R^2$ is hydrogen, $C_{1-6}$ alkyl, aryl or heterocyclyl;
$R^a$ and $R^b$ are, independently, hydrogen or $C_{1-4}$ alkyl; or when $R^2$ is aryl or heterocyclyl $R^a$ may be $C_{2-3}$ alkylene forming a ring with an ortho position on $R^2$;
$R^c$ is hydrogen or hydroxy;

wherein, unless stated otherwise, the foregoing aryl and heterocyclyl moieties are optionally substituted by: halogen, cyano, nitro, hydroxy, oxo, $S(O)_pR^4$, $OC(O)NR^5R^6$, $NR^7R^8$, $NR^9C(O)R^{10}$, $NR^{11}C(O)NR^{12}R^{13}$, $S(O)_2NR^{14}R^{15}$, $NR^{16}S(O)_2R^{17}$, $CON^{18}R^{19}$, $C(O)R^{20}$, $CO_2R^{21}$, $NR^{22}CO_2R^{23}$, $C_{1-6}$ alkyl, $CF_3$, $C_{1-6}$ alkoxy($C_{1-6}$)alkyl, $C_{1-6}$ alkoxy, $OCF_3$, $C_{1-6}$ alkoxy($C_{1-6}$)alkoxy, $C_{1-6}$ alkylthio, $C_{2-6}$ alkenyl, $C_{2-6}$ alkynyl, $C_{3-10}$ cycloalkyl (itself optionally substituted by $C_{1-4}$ alkyl or oxo), methylenedioxy, difluoromethylenedioxy, phenyl, phenyl($C_{1-4}$)alkyl, phenoxy, phenylthio, phenyl($C_{1-4}$)alkoxy, heterocyclyl, heterocyclyl($C_{1-4}$)alkyl, heterocyclyloxy or heterocyclyl($C_{1-4}$)alkoxy; wherein any of the immediately foregoing phenyl and heterocyclyl moieties are optionally substituted with halogen, hydroxy, nitro, $S(O)_q$($C_{1-4}$ alkyl), $S(O)_2NH_2$, $S(O)_2NH$($C_{1-4}$ alkyl), $S(O)_2N$($C_{1-4}$ alkyl$)_2$ (and these alkyl groups may join to form a ring as described for $R^5$ and $R^6$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl$)_2$ (and these alkyl groups may join to form a ring as described for $R^5$ and $R^6$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$;
p and q are, independently, 0, 1 or 2;
$R^3$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{18}$, $R^{19}$,$R^{20}$,$R^{21}$ and $R^{22}$ are, independently, hydrogen, $C_{1-6}$ alkyl (optionally substituted by halogen, hydroxy or $C_{3-10}$ cycloalkyl), $CH_2$($C_{2-6}$ alkenyl), phenyl (itself optionally substituted by halogen, hydroxy, nitro, $NH_2$, NH($C_{1-4}$ alkyl), N($C_{1-4}$ alkyl$)_2$ (and these alkyl groups may join to form a ring as described for $R^5$ and $R^6$ below), $S(O)_2$($C_{1-4}$ alkyl), $S(O)_2NH_2$, $S(O)_2NH$($C_{1-4}$ alkyl), $S(O)_2N$($C_{1-4}$ alkyl$)_2$ (and these alkyl groups may join to form a ring as described for $R^5$ and $R^6$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl$)_2$ (and these alkyl groups may join to form a ring as described for $R^5$ and $R^6$ below), $CO_2H$ $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$) or heterocyclyl (itself optionally substituted by halogen, hydroxy, nitro, $NH_2$, NH($C_{1-4}$ alkyl), N($C_{1-4}$ alkyl$)_2$ (and these alkyl groups may join to form a ring as described for $R^5$ and $R^6$ below), $S(O)_2$($C_{1-4}$ alkyl), $S(O)_2NH_2$, $S(O)_2NH$($C_{1-4}$ alkyl), $S(O)_2N$($C_{1-4}$ alkyl$)_2$ (and these alkyl groups may join to form a ring as described for $R^5$ and $R^6$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl$)_2$ (and these alkyl groups may join to form a ring as described for $R^5$ and $R^6$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$);
alternatively $NR^5R^6$, $NR^7R^8$, $NR^{12}R^{13}$, $NR^{14}R^{15}$, $NR^{18}R^{19}$, may, independently, form a 4-7 membered heterocyclic ring, azetidine, pyrrolidine, piperidine, azepine, morpholine or piperazine, the latter optionally substituted by $C_{1-4}$ alkyl on the distal nitrogen;
$R^4$, $R^{17}$ and $R^{23}$ are, independently, $C_{1-6}$ alkyl (optionally substituted by halogen, hydroxy or $C_{3-10}$ cycloalkyl), $CH_2$($C_{2-6}$ alkenyl), phenyl (itself optionally substituted by halogen, hydroxy, nitro, $NH_2$, NH($C_{1-4}$ alkyl), N($C_{1-4}$ alkyl$)_2$ (and these alkyl groups may join to form a ring as described for $R^5$ and $R^6$ above), $S(O)_2$($C_{1-4}$ alkyl), $S(O)_2NH_2$), $S(O)_2NH$($C_{1-4}$ alkyl), $S(O)_2N$($C_{1-4}$ alkyl$)_2$ (and these alkyl groups may join to form a ring as described for $R^5$ and $R^6$

above), cyano, $C_{1-4}$ alkyl, $C_{1-4}$ alkoxy, $C(O)NH_2$, $C(O)NH(C_{1-4}$ alkyl), $C(O)N(C_{1-4}$ alkyl$)_2$ (and these alkyl groups may join to form a ring as described for $R^5$ and $R^6$ above), $CO_2H$, $CO_2(C_{1-4}$ alkyl), $NHC(O)(C_{1-4}$ alkyl), $NHS(O)_2$ ($C_{1-4}$ alkyl), $C(O)(C_{1-4}$ alkyl), $CF_3$ or $OCF_3$) or heterocyclyl (itself optionally substituted by halogen, hydroxy, nitro, $NH_2$, $NH(C_{1-4}$ alkyl), $N(C_{1-4}$ alkyl$)_2$ (and these alkyl groups may join to form a ring as described for $R^5$ and $R^6$ above), $S(O)_2(C_{1-4}$ alkyl), $S(O)_2NH_2$, $S(O)_2NH(C_{1-4}$ alkyl), $S(O)_2N(C_{1-4}$ akyl$)_2$ (and these alkyl groups may join to form a ring as described for $R^5$ and $R^6$ above), cyano, $C_{1-4}$ alkyl, $C_{1-4}$ alkoxy, $C(O)NH_2$, $C(O)NH(C_{1-4}$ alkyl), $C(O)N(C_{1-4}$ alkyl$)_2$ (and these alkyl groups may join to form a ring as described for $R^5$ and $R^6$ above), $CO_2H$, $CO_2(C_{1-4}$ alkyl), $NHC(O)(C_{1-4}$ alkyl), $NHS(O)_2(C_{1-4}$ alkyl), $C(O)(C_{1-4}$ alkyl), $CF_3$ or $OCF_3$);
aryl is phenyl or naphthyl;
heterocyclyl is furyl, thienyl, pyrrolyl, 2,5-dihydropyrrolyl, thiazolyl, pyrazolyl, oxazolyl, isoxazolyl, imidazolyl, piperidinyl, morpholinyl, pyridinyl, dihydropyridinyl, pyrimidinyl, indolyl, 2,3-dihydroindolyl, benzo[b]furyl, benz[b]thienyl, 2,3-dihydrobenz[b]thienyl, indazolyl, benzimidazolyl, benztriazolyl, benzoxazolyl, benzthiazolyl, 2,3-dihydrobenzthiazolyl, 1,2,3-benzothiadiazolyl, an imidazopyridinyl, thieno[3,2-b]pyridin-6-yl 1,2,3-benzoxadiazolyl, 2,1,3-benzothiadiazolyl, benzofurazan, quinoxalinyl, dihydro-1-benzopyryliumyi, 3,4-dihydro-1H-2,1-benzothiazinyl, a pyrazolopyridine, a purine, quinolinyl, isoquinolinyl, dihydroisoquinolinyl, a naphthyridinyl, a dihydro[1,8]naphthyridinyl, a benzothiazinyl, a dihydrobenzothiazinyl, benzo[d]imidazo[2,1-b]thiazol-2-yl or dibenzothiophenyl;
or an N-oxide thereof; or a pharmaceutically acceptable salt thereof; or a solvate thereof.

**2.** A compound as claimed in claim 1 wherein $R^1$ is phenyl optionally substituted with halogen, $C_{1-4}$ alkyl or $C_{1-4}$ alkoxy.

**3.** A compound as claimed in claim 1 or 2 wherein X is O.

**4.** A compound as claimed in claim 1, 2 or 3 wherein $R^a$ and $R^c$ are both hydrogen.

**5.** A compound as claimed in claim 1, 2, 3 or 4 wherein Z is $CO_2R^b$.

**6.** A compound as claimed in claim 1, 2, 3, 4 or 5 wherein Y is a bond or alkylene (optionally substituted by $C_{1-4}$ alkyl); $R^a$ is hydrogen; and, $R^2$ is hydrogen, $C_{1-6}$ alkyl, phenyl (optionally substituted by halogen, $C_{1-4}$ alkyl, $C_{1-4}$ alkoxy or $NHC(O)(C_{1-4}$ alkyl)) or heterocyclyl (optionally substituted by halogen, $C_{1-4}$ alkyl or $C_{1-4}$ alkoxy).

**7.** A compound as claimed in claim 1, 2, 3, 4 or 5 wherein Y is phenylene (optionally substituted by halogen, $C_{1-4}$ alkyl or $C_{1-4}$ alkoxy) or heterocyclylene (optionally substituted by halogen, $C_{1-4}$ alkyl or $C_{1-4}$ alkoxy); $R^a$ is hydrogen; and $R^2$ is hydrogen or $C_{1-4}$ alkyl.

**8.** A process for preparing a compound of formula (I) as claimed in claim 1, the process comprising:

a) coupling a compound of formula (II):

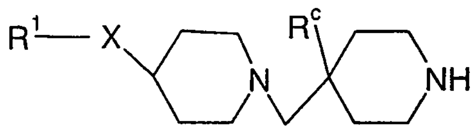

(II)

with a compound of formula (III):

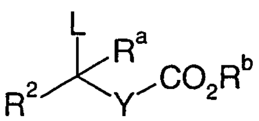

(III)

wherein L is a suitable leaving group;
b) when $R^a$ is hydrogen and Z is $CO_2R^b$, reductive amination of a compound (II) with a compound of formula (IV):

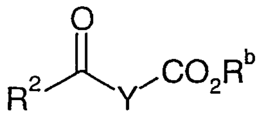 (IV)

wherein $R^b$ is $C_{1-4}$ alkyl, in the presence of $NaBH(OAc)_3$ and acetic acid, or $NaBH_3CN$ in a suitable solvent, optionally followed by hydrolysis of the ester group;

c) when Y is a bond, $R^a$ and $R^b$ are both hydrogen and Z is $CO_2H$, a three component coupling of a compound of formula (II) with compounds of formula (V) and (VI):

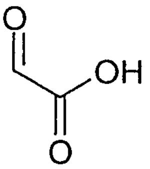 (V) 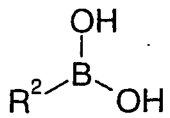 (VI)

in a suitable solvent at a suitable elevated temperature;

d) when Y is a bond and Z is $CO_2H$ performing a nitrile hydrolysis on a compound of formula (XI):

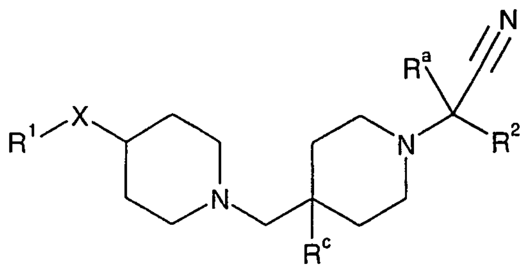 (XI)

e) when Z is tetrazol-5-yl, reacting a compound of formula (XI) with $(CH_3)_3SiN_3$ and $(Bu_3Sn)_2O$ at an elevated temperature;

f) when Z is $NHS(O)_2CF_3$, reacting a compound of formula (XII):

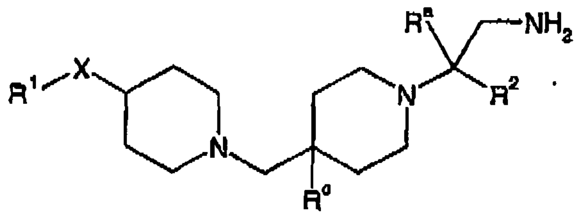 (XII)

with triflic anhydride at a reduced temperature.

**9.** A pharmaceutical composition which comprises a compound of the formula (I), or a pharmaceutically acceptable salt thereof or solvate thereof as claimed in claim 1, and a pharmaceutically acceptable adjuvant, diluent or carrier.

**10.** A compound of the formula (I), or a pharmaceutically acceptable salt thereof or solvate thereof as claimed in claim 1, for use in therapy.

**11.** A compound of formula (I), or a pharmaceutically acceptable salt thereof or solvate thereof as claimed in claim 1, in the manufacture of a medicament for use in therapy.

## Patentansprüche

1. Verbindung der Formel (I):

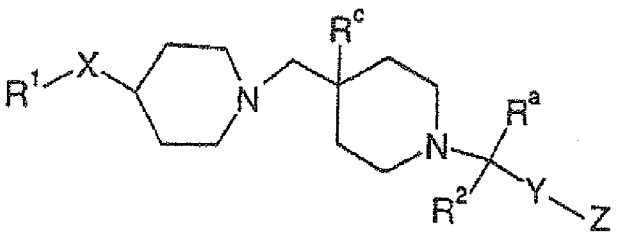

(I)

worin:

X für $CH_2$, C (O) , O, S, S(O), $S(O)_2$ oder $NR^3$ steht;
Y für eine Bindung, $C_{1-6}$-Alkylen (gegebenenfalls substituiert durch $C_{1-4}$-Alkyl oder Phenyl), Phenylen (gegebenenfalls substituiert durch Halogen, Hydroxy, $C_{1-4}$-Alkyl oder $C_{1-4}$-Alkoxy) oder Heterocyclylen (gegebenenfalls substituiert durch Halogen, Hydroxy, $C_{1-4}$-Alkyl oder $C_{1-4}$-Alkoxy) steht;
Z für $CO_2R^b$, NHS (O) $_2CF_3$, S (O) $_2$OH, $OCH_2CO_2R^b$ oder Tetrazolyl steht;
$R^1$ für Wasserstoff, $C_{1-6}$-Alkyl, Aryl oder Heterocyclyl steht;
$R^2$ für Wasserstoff, $C_{1-6}$-Alkyl, Aryl oder Heterocyclyl steht;
$R^a$ und $R^b$ unabhängig voneinander für Wasserstoff oder $C_{1-4}$-Alkyl stehen oder $R^a$ dann, wenn $R^2$ für Aryl oder Heterocyclyl steht, für $C_{2-3}$-Alkylen, das mit einer ortho-Position an $R^2$ einen Ring bildet, stehen kann;
$R^c$ für Wasserstoff oder Hydroxy steht;

wobei die vorstehenden Aryl- und Heterocyclylgruppierungen, sofern nicht anders vermerkt, gegebenenfalls substituiert sind durch: Halogen, Cyano, Nitro, Hydroxy, Oxo, $S(O)_pR^4$, OC (O) $NR^5R^6$, $NR^7R^8$, $NR^9C(O)R^{10}$, NR $^{11}$ C (O) $NR^{12}R^{13}$, S (O) $_2NR^{14}R^{15}$, $NR^{16}$S (O) $_2R^{17}$, C (O) $NR^{18}R^{19}$, C (O) $R^{20}$, $CO_2R^{21}$, $NR^{22}CO_2R^{23}$, $C_{1-6}$-Alkyl, $CF_3$, $C_{1-6}$-Alkoxy-$C_{1-6}$-alkyl, $C_{1-6}$-Alkoxy, $OCF_3$, $C_{1-6}$-Alkoxy-$C_{1-6}$-alkoxy, $C_{1-6}$-Alkylthio, $C_{2-6}$-Alkenyl, $C_{2-6}$-Alkinyl, $C_{3-10}$-Cycloalkyl (selbst gegebenenfalls substituiert durch $C_{1-4}$-Alkyl oder Oxo), Methylendioxy, Difluormethylendioxy, Phenyl, Phenyl-$C_{1-4}$-alkyl, Phenoxy, Phenylthio, Phenyl-$C_{1-4}$-alkoxy, Heterocyclyl, Heterocyclyl-$C_{1-4}$-alkyl, Heterocyclyloxy oder Heterocyclyl-$C_{1-4}$-alkoxy; wobei jede der unmittelbar vorstehenden Phenyl- und Heterocyclylgruppierungen gegebenenfalls durch Halogen, Hydroxy, Nitro, $S(O)_q$($C_{1-4}$-Alkyl), $S(O)_2NH_2$, S $(O)_2$NH ($C_{1-4}$-Alkyl), S $(O)_2$N ($C_{1-4}$-Alkyl)$_2$ (wobei diese Alkylgruppen gemeinsam einen wie unten für $R^5$ und $R^6$ beschriebenen Ring bilden können), Cyano, $C_{1-4}$-Alkyl, $C_{1-4}$-Alkoxy, C (O) $NH_2$, C (O) NH ($C_{1-4}$-Alkyl), C (O) N ($C_{1-4}$-Alkyl)$_2$ (wobei diese Alkylgruppen gemeinsam einen wie unten für $R^5$ und $R^6$ beschriebenen Ring bilden können), $CO_2H$, $CO_2$($C_{1-4}$-Alkyl), NHC(O) ($C_{1-4}$-Alkyl), NHS (O) $_2$ ($C_{1-4}$-Alkyl), C (O) ($C_{1-4}$-Alkyl), $CF_3$ oder $OCF_3$ substituiert ist;
p und q unabhängig voneinander für 0, 1 oder 2 stehen;
$R^3$ , $R^5$ , $R^6$ , $R^7$ , $R^8$ , $R^9$ , $R^{10}$ , $R^{11}$ , $R^{12}$ , $R^{13}$ , $R^{14}$ , $R^{15}$ $R^{16}$, $R^{18}$, $R^{19}$, $R^{20}$, $R^{21}$ und $R^{22}$ unabhängig voneinander für Wasserstoff, $C_{1-6}$-Alkyl (gegebenenfalls substituiert durch Halogen, Hydroxy oder $C_{3-10}$-Cycloalkyl), $CH_2$($C_{2-6}$-Alkenyl), Phenyl (selbst gegebenenfalls substituiert durch Halogen, Hydroxy, Nitro, $NH_2$, NH ($C_{1-4}$-Alkyl), N ($C_{1-4}$-Alkyl) $_2$ (wobei diese Alkylgruppen gemeinsam einen wie unten für $R^5$ und $R^6$ beschriebenen Ring bilden können), S $(O)_2$($C_{1-4}$-Alkyl), S $(O)_2NH_2$, S (O) $_2$NH ($C_{1-4}$-Alkyl) , S $(O)_2$N ($C_{1-4}$-Alkyl)$_2$ (wobei diese Alkylgruppen gemeinsam einen wie unten für $R^5$ und $R^6$ beschriebenen Ring bilden können), Cyano, $C_{1-4}$-Alkyl, $C_{1-4}$-Alkoxy, C $(O)NH_2$, C(O)NH($C_{1-4}$-Alkyl), C(O)N ($C_{1-4}$-Alkyl)$_2$ (wobei diese Alkylgruppen gemeinsam einen wie unten für $R^5$ und $R^6$ beschriebenen Ring bilden können), $CO_2H$, $CO_2$($C_{1-4}$-Alkyl), NHC(O) ($C_{1-4}$-Alkyl), NHS $(O)_2$($C_{1-4}$-Alkyl), C (O)($C_{1-4}$-Alkyl), $CF_3$ oder $OCF_3$) oder Heterocyclyl (selbst gegebenenfalls substituiert durch Halogen, Hydroxy, Nitro, $NH_2$, NH($C_{1-4}$-Alkyl) , N($C_{1-4}$-Alkyl) $_2$ (wobei diese Alkylgruppen gemeinsam einen wie unten für $R^5$ und $R^6$ beschriebenen Ring bilden können), S (O) $_2$ ($C_{1-4}$-Alkyl), $S(O)_2NH_2$, S (O) $_2$NH ($C_{1-4}$-Alkyl), S $(O)_2$N ($C_{1-4}$-Alkyl) $_2$ (wobei diese Alkylgruppen gemeinsam einen wie unten für $R^5$ und $R^6$ beschriebenen Ring bilden können), Cyano, $C_{1-4}$-Alkyl, $C_{1-4}$-Alkoxy, $C(O)NH_2$, C (O) NH ($C_{1-4}$-Alkyl), C (O) N ($C_{1-4}$-Alkyl) $_2$ (wobei diese Alkylgruppen gemeinsam einen wie unten für $R^5$ und $R^6$ beschriebenen Ring bilden können), $CO_2H$, $CO_2$ ($C_{1-4}$-Alkyl), NHC (O) ($C_{1-4}$-Alkyl), NHS (O) $_2$ ($C_{1-4}$-Alkyl), C(O) ($C_{1-4}$-Alkyl), $CF_3$ oder $OCF_3$) stehen;
alternativ dazu $NR^5R^6$, $NR^7R^8$, $NR^{12}R^{13}$, $NR^{14}R^{15}$, $NR^{18}R^{19}$ unabhängig voneinander einen 4-7-gliedrigen heterocyclischen Ring, Azetidin, Pyrrolidin, Piperidin, Azepin, Morpholin oder Piperazin bilden können, wobei letzteres gegebenenfalls am distalen Stickstoff durch $C_{1-4}$-Alkyl substituiert ist;

$R^4$, $R^{17}$ und $R^{23}$ unabhängig voneinander für $C_{1-6}$-Alkyl (gegebenenfalls substituiert durch Halogen, Hydroxy oder $C_{3-10}$-Cycloalkyl), $CH_2$ ($C_{2-6}$-Alkenyl), Phenyl (selbst gegebenenfalls substituiert durch Halogen, Hydroxy, Nitro, $NH_2$, NH ($C_{1-4}$-Alkyl), N ($C_{1-4}$-Alkyl)$_2$ (wobei diese Alkylgruppen gemeinsam einen wie oben für $R^5$ und $R^6$ beschriebenen Ring bilden können), S (O) $_2$ ($C_{1-4}$-Alkyl), S $(O)_2NH_2$, S (O) $_2$NH ($C_{1-4}$-Alkyl), S $(O)_2$N ($C_{1-4}$-Alkyl)$_2$ (wobei diese Alkylgruppen gemeinsam einen wie oben für $R^5$ und $R^6$ beschriebenen Ring bilden können), Cyano, $C_{1-4}$-Alkyl, $C_{1-4}$-Alkoxy, $C(O)NH_2$, C(O) NH ($C_{1-4}$-Alkyl), C (0) N ($C_{1-4}$-Alkyl) $_2$ (wobei diese Alkylgruppen gemeinsam einen wie oben für $R^5$ und $R^6$ beschriebenen Ring bilden können), $CO_2H$, $CO_2$($C_{1-4}$-Alkyl), NHC (O) ($C_{1-4}$-Alkyl), NHS (O) $_2$($C_{1-4}$-Alkyl), C(O)($C_{1-4}$-Alkyl) , $CF_3$ oder $OCF_3$) oder Heterocyclyl (selbst gegebenenfalls substituiert durch Halogen, Hydroxy, Nitro, $NH_2$, NH ($C_{1-4}$-Alkyl), N ($C_{1-4}$-Alkyl)$_2$ (wobei diese Alkylgruppen gemeinsam einen wie oben für $R^5$ und $R^6$ beschriebenen Ring bilden können), S $(O)_2$($C_{1-4}$-Alkyl) , S $(O)_2NH_2$, S (O) $_2$NH ($C_{1-4}$-Alkyl), S $(O)_2$N ($C_{1-4}$-Alkyl) $_2$ (wobei diese Alkylgruppen gemeinsam einen wie oben für $R^5$ und $R^6$ beschriebenen Ring bilden können), Cyano, $C_{1-4}$-Alkyl, $C_{1-4}$-Alkoxy, $C(O)NH_2$, C (O) NH ($C_{1-4}$-Alkyl), C (0) N ($C_{1-4}$-Alkyl) $_2$ (wobei diese Alkylgruppen gemeinsam einen wie oben für $R^5$ und $R^6$ beschriebenen Ring bilden können), $CO_2H$, $CO_2$($C_{1-4}$-Alkyl), NHC (0) ($C_{1-4}$-Alkyl), NHS $(O)_2$($C_{1-4}$-Alkyl) , C(O) ($C_{1-4}$-Alkyl), $CF_3$ oder $OCF_3$) stehen;
Aryl für Phenyl oder Naphthyl steht;
Heterocyclyl für Furyl, Thienyl, Pyrrolyl, 2,5-Dihydropyrrolyl, Thiazolyl, Pyrazolyl, Oxazolyl, Isoxazolyl, Imidazolyl, Piperidinyl, Morpholinyl, Pyridinyl, Dihydropyridinyl, Pyrimidinyl, Indolyl, 2,3-Dihydroindolyl, Benzo[b]furyl, Benz[b]thienyl, 2,3-Dihydrobenz[b]thienyl, Indazolyl, Benzimidazolyl, Benztriazolyl, Benzoxazolyl, Benzthiazolyl, 2,3-Dihydrobenzthiazolyl, 1,2,3-Benzothiadiazolyl, ein Imidazopyridinyl, Thieno[3,2-b]pyridin-6-yl, 1,2,3-Benzoxadiazolyl, 2,1,3-Benzothiadiazolyl, Benzofurazan, Chinoxalinyl, Dihydro-1-benzopyryliumyl, 3,4-Dihydro-1H-2,1-benzothiazinyl, ein Pyrazolopyridin, ein Purin, Chinolinyl, Isochinolinyl, Dihydroisochinolinyl, ein Naphthyridinyl, ein Dihydro [1,8]naphthyridinyl, ein Benzothiazinyl, ein Dihydrobenzothiazinyl, Benzo[d]imidazo[2,1-b]thiazol-2-yl oder Dibenzothiophenyl steht;
oder ein N-Oxid davon oder ein pharmazeutisch annehmbares Salz davon oder ein Solvat davon.

**2.** Verbindung nach Anspruch 1, in der $R^1$ für gegebenenfalls durch Halogen, $C_{1-4}$-Alkyl oder $C_{1-4}$-Alkoxy substituiertes Phenyl steht.

**3.** Verbindung nach Anspruch 1 oder 2, in der X für 0 steht.

**4.** Verbindung nach Anspruch 1, 2 oder 3, in der $R^a$ und $R^c$ beide für Wasserstoff stehen.

**5.** Verbindung nach Anspruch 1, 2, 3 oder 4, in der 2 für $CO_2R^b$ steht.

**6.** Verbindung nach Anspruch 1, 2, 3, 4 oder 5, in der Y für eine Bindung oder Alkylen (gegebenenfalls substituiert durch $C_{1-4}$-Alkyl) steht; $R^a$ für Wasserstoff steht und $R^2$ für Wasserstoff, $C_{1-6}$-Alkyl, Phenyl (gegebenenfalls substituiert durch Halogen, $C_{1-4}$-Alkyl, $C_{1-4}$-Alkoxy oder NHC(O) ($C_{1-4}$-Alkyl)) oder Heterocyclyl (gegebenenfalls substituiert durch Halogen, $C_{1-4}$-Alkyl oder $C_{1-4}$-Alkoxy) steht.

**7.** Verbindung nach Anspruch 1, 2, 3, 4 oder 5, in der Y für Phenylen (gegebenenfalls substituiert durch Halogen, $C_{1-4}$-Alkyl oder $C_{1-4}$-Alkoxy) oder Heterocyclylen (gegebenenfalls substituiert durch Halogen, $C_{1-4}$-Alkyl oder $C_{1-4}$-Alkoxy) steht; $R^a$ für Wasserstoff steht und $R^2$ für Wasserstoff oder $C_{1-4}$-Alkyl steht.

**8.** Verfahren zur Herstellung einer Verbindung der Formel (I) nach Anspruch 1, bei dem man:

a) eine Verbindung der Formel (II):

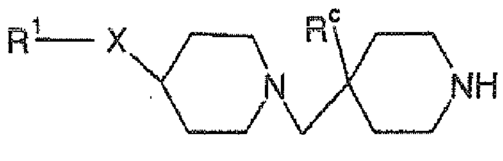 (II)

mit einer Verbindung der Formel (III):

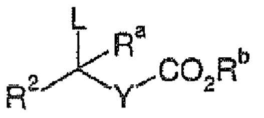

(III)

worin L für eine geeignete Abgangsgruppe steht, kuppelt;

b) wenn $R^a$ für Wasserstoff steht und Z für $CO_2R^b$ steht, eine Verbindung (II) in Gegenwart von $NaBH(OAc)_3$ und Essigsäure oder $NaBH_3CN$ in einem geeigneten Lösungsmittel mit einer Verbindung der Formel (IV):

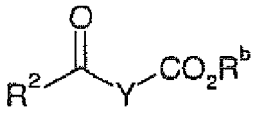

(IV)

worin $R^b$ für $C_{1-4}$-Alkyl steht, reduktiv aminiert und gegebenenfalls danach die Estergruppe hydrolysiert;

c) wenn Y für eine Bindung steht, $R^a$ und $R^b$ beide für Wasserstoff stehen und Z für $CO_2H$ steht, eine Dreikomponentenkupplung einer Verbindung der Formel (II) mit Verbindungen der Formel (V) und (VI) :

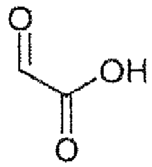

(V)

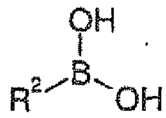

(VI)

in einem geeigneten Lösungsmittel bei einer geeigneten erhöhten Temperatur durchführt;

d) wenn Y für eine Bindung steht und Z für $CO_2H$ steht, an einer Verbindung der Formel (XI):

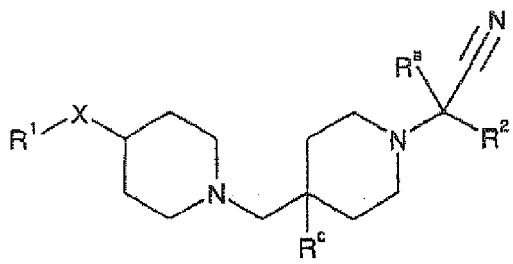

(XI)

eine Nitrilhydrolyse durchführt;

e) wenn Z für Tetrazol-5-yl steht, eine Verbindung der Formel (XI) bei erhöhter Temperatur mit $(CH_3)_3SiN_3$ und $(Bu_3Sn)_2O$ umsetzt;

f) wenn Z für $NHS(O)_2CF_3$ steht, eine Verbindung der Formel (XII) bei verringerter Temperatur mit Trifluormethansulfonsäureanhydrid umsetzt.

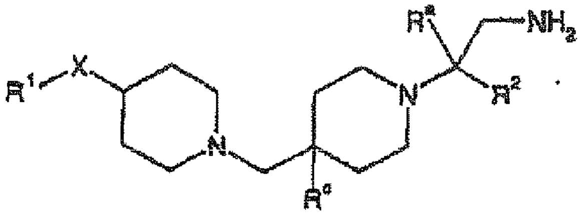

(XII)

**9.** Pharmazeutische Zusammensetzung, die eine Verbindung der Formel (I) oder ein pharmazeutisch annehmbares Salz davon oder Solvat davon nach Anspruch 1 und einen pharmazeutisch annehmbaren Hilfsstoff, ein pharmazeutisch annehmbares Verdünnungsmittel oder einen pharmazeutisch annehmbaren Träger enthält.

**10.** Verbindung der Formel (I) oder ein pharmazeutisch annehmbares Salz davon oder Solvat davon nach Anspruch 1 zur Verwendung bei der Therapie.

**11.** Verbindung der Formel (I) oder ein pharmazeutisch annehmbares Salz davon oder Solvat davon nach Anspruch 1 bei der Herstellung eines Arzneimittels zur Verwendung bei der Therapie.

**Revendications**

**1.** Composé de formule (I) :

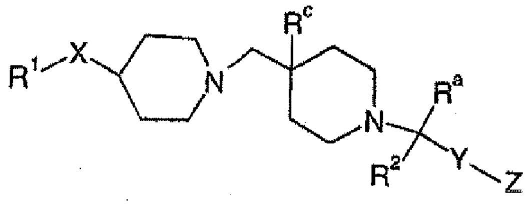

(I)

dans laquelle :

X est $CH_2$, C(O), O, S, S (O), S $(O)_2$ ou $NR^3$ ;
Y est une liaison, $C_{1-6}$ alkylène (éventuellement substitué par $C_{1-4}$ alkyle ou phényle), phénylène (éventuellement substitué par halogène, hydroxy, $C_{1-4}$ alkyle ou $C_{1-4}$ alcoxy) ou hétérocyclylène (éventuellement substitué par halogène, hydroxy, $C_{1-4}$ alkyle ou $C_{1-4}$ alcoxy);
Z est $CO_2R^b$, $NHS(O)_2CF_3$, $S(O)_2OH$, $OCH_2CO_2R^b$ ou tétrazolyle ;
$R^1$ est hydrogène, $C_{1-6}$ alkyle, aryle ou hétérocyclyle ;
$R^2$ est hydrogène, $C_{1-6}$ alkyle, aryle ou hétérocyclyle ;
$R^a$ et $R^b$ sont, indépendamment, hydrogène ou $C_{1-4}$ alkyle ; ou lorsque $R^2$ est aryle ou hétérocyclyle $R^a$ peut être $C_{2-3}$ alkylène formant un cycle avec une position ortho sur $R^2$ ;
$R^c$ est hydrogène ou hydroxy ;

où, sauf indication contraire, les motifs aryle et hétérocyclyle précédents sont éventuellement substitués par: halogène, cyano, nitro, hydroxy, oxo, $S(O)_pR^4$, $OC(O)NR^5R^6$, $NR^7R^8$, $NR^9C(O)R^{10}$, $NR^{11}C(O)NR^{12}R^{13}$, $S(O)_2NR^{14}R^{15}$, $NR^{16}S(O)_2R^{17}$, C (O) $NR^{18}R^{19}$, $C(O)R^{20}$, $CO_2R^{21}$, $NR^{22}CO_2R^{23}$, $C_{1-6}$ alkyle, $CF_3$, $C_{1-6}$ alcoxy ($C_{1-6}$) alkyle, $C_{1-6}$ alcoxy, $OCF_3$, $C_{1-6}$ alcoxy ($C_{1-6}$) alcoxy, $C_{1-6}$ alkylthio, $C_{2-6}$ alcényle, $C_{2-6}$ alkynyle, $C_{3-10}$ cycloalkyle (lui-même éventuellement substitué par $C_{1-4}$ alkyle ou oxo), méthylènedioxy, difluorométhylènedioxy, phényle, phényl ($C_{1-4}$) alkyle, phénoxy, phénylthio, phényl ($C_{1-4}$) alcoxy, hétérocyclyle, hétérocyclyl($C_{1-4}$)alkyle, hétérocyclyloxy ou hétérocyclyl ($C_{1-4}$) alcoxy ; où l'un quelconque des motifs phényle et hétérocyclyle qui précèdent immédiatement sont éventuellement substitués par halogène, hydroxy, nitro, S $(O)_q$($C_{1-4}$ alkyle), S (O) $_2NH_2$, S (O) $_2$NH ($C_{1-4}$ alkyle), S $(O)_2$N ($C_{1-4}$ alkyle) $_2$ (et ces groupements alkyle peuvent s'unir pour former un cycle comme décrit pour $R^5$ et $R^6$ ci-dessous), cyano, $C_{1-4}$ alkyle, $C_{1-4}$ alcoxy, $C(O)NH_2$, C(O)NH($C_{1-4}$alkyle), C(O)N($C_{1-4}$ alkyle) $_2$ (et ces groupements alkyle peuvent s'unir pour former un cycle comme décrit pour $R^5$ et $R^6$ ci-dessous), $CO_2H$, $CO_2$ ($C_{1-4}$ alkyle), NHC (O) ($C_{1-4}$ alkyle), $NHS(O)_2$($C_{1-4}$ alkyle), C(O)($C_{1-4}$ alkyl), $CF_3$ ou $OCF_3$;
p et q sont, indépendamment, 0, 1 ou 2 ;
$R^3$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{18}$, $R^{19}$, $R^{20}$, $R^{21}$ et $R^{22}$ sont, indépendamment, hydrogène, $C_{1-6}$ alkyle (éventuellement substitué par halogène, hydroxy ou $C_{3-10}$ cycloalkyle), $CH_2$ ($C_{2-6}$ alcényle), phényle (lui-même éventuellement substitué par halogène, hydroxy, nitro, $NH_2$, NH ($C_{1-4}$ alkyle), N ($C_{1-4}$ alkyle) $_2$ (et ces groupements alkyle peuvent s'unir pour former un cycle comme décrit pour $R^5$ et $R^6$ ci-dessous), S (O) $_2$ ($C_{1-4}$ alkyle), S (O) $_2NH_2$, S (O) $_2$NH ($C_{1-4}$ alkyle), S (O) $_2$N ($C_{1-4}$ alkyle)$_2$ (et ces groupements alkyle peuvent s'unir pour former un cycle comme décrit pour $R^5$ et $R^6$ ci-dessous), cyano, $C_{1-4}$ alkyle, $C_{1-4}$ alcoxy, C(0)$NH_2$, C(O)NH ($C_{1-4}$ alkyle), C (O) N ($C_{1-4}$ alkyle)$_2$ (et ces groupements alkyle peuvent s'unir pour former un cycle comme décrit pour $R^5$ et $R^6$ ci-dessous), $CO_2H$, $CO_2$ ($C_{1-4}$ alkyle), NHC (O) ($C_{1-4}$ alkyle), NHS (O) $_2$ ($C_{1-4}$ alkyle), C (O) ($C_{1-4}$ alkyle),

$CF_3$ ou $OCF_3$) ou hétérocyclyle (lui-même éventuellement substitué par halogène, hydroxy, nitro, $NH_2$, NH($C_{1-4}$ alkyle), N ($C_{1-4}$ alkyle) $_2$ (et ces groupements alkyle peuvent s'unir pour former un cycle comme décrit pour $R^5$ et $R^6$ ci-dessous), S (O) $_2$ ($C_{1-4}$ alkyle), S (O) $_2NH_2$, S (O) $_2$NH ($C_{1-4}$ alkyle), S (O) $_2$N ($C_{1-4}$ alkyle)$_2$ (et ces groupements alkyle peuvent s'unir pour former un cycle comme décrit pour $R^5$ et $R^6$ ci-dessous), cyano, $C_{1-4}$ alkyle, $C_{1-4}$ alcoxy, C(O)$NH_2$, C (O) NH ($C_{1-4}$ alkyle), C (O) N ($C_{1-4}$ alkyle)$_2$ (et ces groupements alkyle peuvent s'unir pour former un cycle comme décrit pour $R^5$ et $R^6$ ci-dessous), $CO_2$H, $CO_2$($C_{1-4}$ alkyle), NHC(O) ($C_{1-4}$ alkyle), NHS (O) $_2$ ($C_{1-4}$ alkyle), C (O) ($C_{1-4}$ alkyle), $CF_3$ ou $OCF_3$);

de manière alternative $NR^5R^6$, $NR^7R^8$, $NR^{12}R^{13}$, $NR^{14}R^{15}$, $NR^{18}R^{19}$ peuvent, indépendamment, former un cycle hétérocyclique à 4-7 chaînons, azétidine, pyrrolidine, pipéridine, azépine, morpholine ou pipérazine, cette dernière éventuellement substituée par $C_{1-4}$ alkyle sur l'azote distal ;

$R^4$, $R^{17}$ et $R^{23}$ sont, indépendamment, $C_{1-6}$ alkyle (éventuellement substitué par halogène, hydroxy ou $C_{3-10}$ cycloalkyle), $CH_2$($C_{2-6}$ alcényl), phényle (lui-même éventuellement substitué par halogène, hydroxy, nitro, $NH_2$, NH ($C_{1-4}$ alkyle), N ($C_{1-4}$ alkyle)$_2$ (et ces groupements alkyle peuvent s'unir pour former un cycle comme décrit pour $R^5$ et $R^6$ ci-dessus), S (O)$_2$($C_{1-4}$ alkyle), S (O)$_2NH_2$, S (O)$_2$NH ($C_{1-4}$ alkyle), S (O)$_2$N ($C_{1-4}$ alkyle) $_2$ (et ces groupements alkyle peuvent s'unir pour former un cycle décrit pour $R^5$ et $R^6$ ci-dessus), cyano, $C_{1-4}$ alkyle, $C_{1-4}$ alcoxy, C(O) $NH_2$, C(O) NH ($C_{1-4}$ alkyle) , C(O) N ($C_{1-4}$ alkyle)$_2$ (et ces groupements alkyle peuvent s'unir pour former un cycle comme décrit pour $R^5$ et $R^6$ ci-dessus), $CO_2$H, $CO_2$ ($C_{1-4}$ alkyle), NHC (O) ($C_{1-4}$ alkyle), NHS (O)$_2$ ($C_{1-4}$ alkyle) , C (O) ($C_{1-4}$ alkyle) , $CF_3$ ou $OCF_3$) ou hétérocyclyle (lui-même éventuellement substitué par halogène, hydroxy, nitro, $NH_2$, NH ($C_{1-4}$ alkyle), N ($C_{1-4}$ alkyle) $_2$ (et ces groupements alkyle peuvent s'unir pour former un cycle comme décrit pour $R^5$ et $R^6$ ci-dessus), S (O)$_2$($C_{1-4}$ alkyle) , S (O)$_2NH_2$, S (O) $_2$NH ($C_{1-4}$ alkyle), S (O) $_2$N ($C_{1-4}$ alkyle) $_2$ (et ces groupements alkyle peuvent s'unir pour former un cycle comme décrit pour $R^5$ et $R^6$ ci-dessus), cyano, $C_{1-4}$ alkyle, $C_{1-4}$ alcoxy, C(O)$NH_2$, C (O) NH ($C_{1-4}$ alkyle), C (O) N ($C_{1-4}$ alkyle)$_2$ (et ces groupements alkyle peuvent s'unir pour former un cycle comme décrit pour $R^5$ et $R^6$ ci-dessus), $CO_2$H, $CO_2$ ($C_{1-4}$ alkyle), NHC (O) ($C_{1-4}$ alkyle), NHS (O)$_2$ ($C_{1-4}$ alkyle) , C (O) ($C_{1-4}$ alkyle), $CF_3$ ou $OCF_3$) ;

aryle est phényle ou naphtyle ;

hétérocyclyle est furyle, thiényle, pyrrolyle, 2,5-dihydropyrrolyle, thiazolyle, pyrazolyle, oxazolyle, isoxazolyle, imidazolyle, pipéridinyle, morpholinyle, pyridinyle, dihydropyridinyle, pyrimidinyle, indolyle, 2,3-dihydroindolyle, benzo [b]furyle, benz[b]thiényle, 2,3-dihydrobenz[b]thiényle, indazolyle, benzimidazolyle, benztriazolyle, benzoxazolyle, benzthiazolyle, 2,3-dihydrobenzthiazolyle, 1,2,3-benzothiadiazolyl, un imidazopyridinyle, thiéno[3,2-b]pyridin-6-yle, 1,2,3-benzoxadiazolyle, 2,1,3-benzothiadiazolyle, benzofurazan, quinoxalinyle, dihydro-1-benzopyryliumyle, 3,4-dihydro-1H-2,1-benzothiazinyle, une pyrazolopyridine, une purine, quinoléinyle, isoquinoléinyle, dihydroisoquinoléinyle, un naphtyridinyle, un dihydro[1,8]naphtyridinyle, un benzothiazinyle, un dihydrobenzothiazinyle, un benzo [d]imidazo[2,1-b]thiazol-2-yle ou dibenzothiophényle ;

ou un N-oxyde de celui-ci ; ou un sel pharmaceutiquement acceptable de celui-ci ; ou un solvate de celui-ci.

2. Composé selon la revendication 1, **caractérisé en ce que** $R^1$ est phényle éventuellement substitué par halogène, $C_{1-4}$ alkyle ou $C_{1-4}$ alcoxy.

3. Composé selon la revendication 1 ou 2, **caractérisé en ce que** X est O.

4. Composé selon la revendication 1, 2 ou 3, **caractérisé en ce que** $R^a$ et $R^c$ sont tous deux hydrogène.

5. Composé selon la revendication 1, 2, 3 ou 4, **caractérisé en ce que** Z est $CO_2R^b$.

6. Composé selon la revendication 1, 2, 3, 4 ou 5, **caractérisé en ce que** Y est une liaison ou alkylène (éventuellement substitué par $C_{1-4}$ alkyle) ; $R^a$ est hydrogène ; et $R^2$ est hydrogène, $C_{1-6}$ alkyle, phényle (éventuellement substitué par halogène, $C_{1-4}$ alkyle, $C_{1-4}$ alcoxy ou NHC (O) ($C_{1-4}$ alkyle)) ou hétérocyclyle (éventuellement substitué par halogène, $C_{1-4}$ alkyle ou $C_{1-4}$ alcoxy).

7. Composé selon la revendication 1, 2, 3, 4 ou 5, **caractérisé en ce que** Y est phénylène (éventuellement substitué par halogène, $C_{1-4}$ alkyle ou $C_{1-4}$ alcoxy) ou hétérocyclylène (éventuellement substitué par halogène, $C_{1-4}$ alkyle ou $C_{1-4}$ alcoxy); $R^a$ est hydrogène; et $R^2$ est hydrogène ou $C_{1-4}$ alkyle.

8. Procédé de préparation d'un composé de formule (I) selon la revendication 1, le procédé comprenant:

a) le couplage d'un composé de formule (II) :

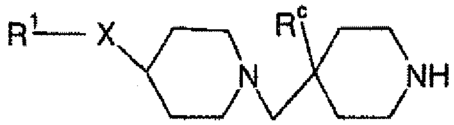

(II)

avec un composé de formule (III) :

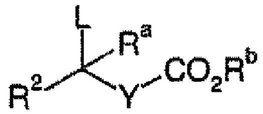

(III)

dans laquelle L est un groupement partant convenable ;

b) lorsque $R^a$ est hydrogène et Z est $CO_2R^b$, une amination réductrice d'un composé (II) avec un composé de formule (IV) :

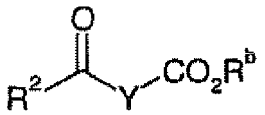

(IV)

dans laquelle $R^b$ est $C_{1-4}$ alkyle, en présence de $NaBH(OAc)_3$ et d'acide acétique, ou de $NaBH_3CN$ dans un solvant convenable, éventuellement suivie d'hydrolyse du groupement ester ;

c) lorsque Y est une liaison, $R^a$ et $R^b$ sont tous deux hydrogène et Z est $CO_2H$, un couplage à trois composants d'un composé de formule (II) avec des composés de formules (V) et (VI) :

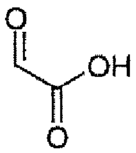

(V)

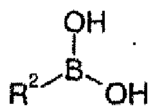

(VI)

dans un solvant convenable, à une température élevée convenable ;

d) lorsque Y est une liaison et Z est $CO_2H$, la mise en oeuvre d'une hydrolyse de nitrile sur un composé de formule (XI):

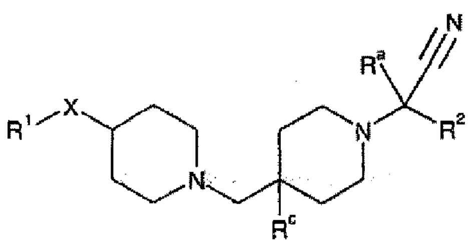

(XI)

e) lorsque Z est tétrazol-5-yle, la réaction d'un composé de formule (XI) avec $(CH_3)_3SiN_3$ et $(Bu_3Sn)_2O$ à une température élevée ;

f) lorsque Z est $NHS(O)_2CF_3$, la réaction d'un composé de formule (XII) :

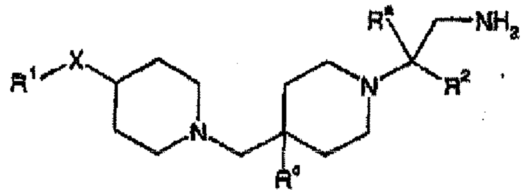

(XII)

avec de l'anhydride triflique à une température réduite.

**9.** Composition pharmaceutique comprenant un composé de formule (I), ou un sel pharmaceutiquement acceptable de celui-ci ou un solvate de celui-ci selon la revendication 1, et un adjuvant, diluant ou support pharmaceutiquement acceptable.

**10.** Composé de formule (I), ou un sel pharmaceutiquement acceptable de celui-ci ou un solvate de celui-ci selon la revendication, destiné à être utilisé en thérapie.

**11.** Composé de formule (I), ou un sel pharmaceutiquement acceptable de celui-ci ou un solvate de celui-ci selon la revendication 1, dans la fabrication d'un médicament destiné à être utilisé en thérapie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9938514 A
- WO 9904794 A
- WO 0035877 A
- WO 0177101 A
- WO 0000488 A
- WO 9801425 A
- WO 9806697 A
- WO 02081449 A
- EP 1076055 A1
- WO 0066559 A


### Non-patent literature cited in the description


- **GREIFF L et al.** Experimental common cold increase mucosal output of eotaxin in atopic individuals. *Allergy,* 1999, vol. 54 (11), 1204-8
- **KAWAGUCHI M et al.** Expression of eotaxin by normal airway epithelial cells after virus A infection. *Int. Arch. Allergy Immunol.,* 2000, vol. 122, S1 44
- **HANSEL et al.** *J. Immunol. Methods,* 1991, vol. 145, 105-110
- **STRATH et al.** *J. Immunol. Methods,* 1985, vol. 83, 209
- **HARRISON, R.W.S. ; CARSWELL, H. ; YOUNG, J.M.** *European J. Pharmacol.,* 1984, vol. 106, 405-409